# EUROPEAN PATENT SPECIFICATION

(11) **EP 2 364 317 B1**
(45) Date of publication and mention of the grant of the patent: **29.07.2015**
(21) Application number: 09805379.6
(22) Date of filing: 31.07.2009
(51) Int. Cl.: A61P 25/28, C07D 221/18, A61K 9/00

(54) **DOPAMINE RECEPTOR LIGANDS WITH ENHANCED DURATION OF ACTION**
DOPAMINREZEPTORLIGANDEN MIT VERBESSERTER WIRKUNGSDAUER
LIGANDS DE RÉCEPTEUR DE DOPAMINE À DURÉE D'ACTION PROLONGÉE

(30) Priority: 05.08.2008 US 86398 P
(43) Date of publication of application: 14.09.2011
(73) Proprietor: Effipharma, Inc., Chapel Hill, NC 27514 (US)
(72) Inventor: MAILMAN, Richard, B., Hershey, PA 17033-0850 (US)
(74) Representative: Wilson, Gary
(86) International application number: PCT/US2009/052334
(87) International publication number: WO 2010/017093

(56) References cited:
- WO-A1-93/24462
- WO-A1-96/02513
- WO-A2-2005/062894
- WO-A2-2006/012640
- CUEVA JUAN PABLO ET AL: "trans-2,3-dihydroxy-6a,7,8,12b-tetrahydro -6H-chromeno[3,4-c]isoquino line: Synthesis, resolution, and preliminary pharmacological characterization of a new dopamine D-1 receptor full agonist", JOURNAL OF MEDICINAL CHEMISTRY, AMERICAN CHEMICAL SOCIETY, US, vol. 49, no. 23, 1 November 2006 (2006-11-01), pages 6848-6857, XP002480375, ISSN: 0022-2623, DOI: 10.1021/JM0604979
- TIMM A KNOERZER: "Synthesis and Biological Evaluation of a Series of Substituted Benzo[a]phenanthridines as Agonists at D1 and D2 Dopamine Receptors", JOURNAL OF MEDICINAL CHEMISTRY, AMERICAN CHEMICAL SOCIETY, US, vol. 38, no. 16, 1 August 1995 (1995-08-01), pages 3062-3070, XP008140741, ISSN: 0022-2623, DOI: 10.1021/JM00016A009
- TIMM A. KNOERZER: 'Synthesis and Biological Evaluation of a Series of Substituted Benzo[a]phenanthridines as Agonists at D1 and D2 Dopamine Receptors' J.MED.CHEM. vol. 38, 1995, pages 3062 - 3070, XP008140741

## Description

### FIELD OF THE INVENTION

The present invention relates to novel ligands for dopamine receptors, in particular, the dopamine D₁ receptor. More particularly, this invention is directed to certain substituted transhexahydrobenzo[a]phenanthridine and related compounds useful as selective D₁ dopamine receptor agonists, with relatively long duration of action and a relatively low likelihood of developing tolerance. The compounds can be used to treat dopamine-related dysfunction of the central nervous system and selected peripheral systems.

This invention was made with Government support under Grant No. MH-40537 awarded by the National Institute of Health. The Government has relinquished rights in the invention.

This application claims priority to U.S. Provisional Application 61/086,398, the contents of which are hereby incorporated by reference for all purposes.

### BACKGROUND OF THE INVENTION

Dopamine receptors are divided into two pharmacological families labeled as D₁ and D₂ (Garau et al., 1978; Kebabian and Calne, 1979) that are now known to be coded by five genes, the products of which are all members of what is termed either the 7 transmembrane (7TM) or G protein-coupled receptor (GPCR) superfamily. The D₂-like family is coded by three different genes yielding D₂, D₃ and D₄ receptors. The other two genes code for the D₁ and D₅ (Dearry et al., 1990; Monsma et al., 1990; Sunahara et al., 1990; Zhou et al., 1990) (Sunahara et al., 1991; Tiberi et al., 1991), the ones of immediate importance to this application. The dopamine receptors have been the subject of numerous reviews and books (Huang et al., 2001; Jenner and Demirdemar, 1997; Neve and Neve, 1997; Neve et al., 2004; Sealfon and Olanow, 2000). For the purposes herein, references to D₁ or D₂ agonist shall refer to actions at the pharmacological subclasses (e.g., D₁ means the D₁-like receptors D₁ and D₅; and D₂ means the D₂-like D₂, D₃, and D₄) unless otherwise specified.

First described in the early 19th century (Parkinson, 1817), PD is the disorder with the clearest link to dopamine dysfunction. The motor effects are due principally to degeneration of the dopaminergic cells in the substantia nigra pars compacta, with consequent loss of dopamine terminals in the striatum. Numerous etiological mechanisms have been

### FIELD OF THE INVENTION

The present invention relates to the use of 2-methyl dihydrexidine to treat parkinsonism type disesases.

This invention was made with Government support under Grant No. MH-40537 awarded by the National Institute of Health. The Government has relinquished rights in the invention.

### BACKGROUND OF THE INVENTION

Dopamine receptors are divided into two pharmacological families labeled as D₁ and D₂ (Garau et al., 1978; Kebabian and Calne, 1979) that are now known to be coded by five genes, the products of which are all members of what is termed either the 7 transmembrane (7TM) or G protein-coupled receptor (GPCR) superfamily. The D₂-like family is coded by three different genes yielding D₂, D₃ and D₄ receptors. Of

### FIELD OF THE INVENTION

The present invention relates to novel ligands for dopamine receptors, in particular, the dopamine D₁ receptor. More particularly, this invention is directed to certain substituted transhexahydrobenzo[a]phenanthridine and related compounds useful as selective D₁ dopamine receptor agonists, with relatively long duration of action and a relatively low likelihood of developing tolerance. The compounds can be used to treat dopamine-related dysfunction of the central nervous system and selected peripheral systems.

This invention was made with Government support under Grant No. MH-40537 awarded by the National Institute of Health. The Government has relinquished rights in the invention.

### BACKGROUND OF THE INVENTION

Dopamine receptors are divided into two pharmacological families labeled as D₁ and D₂ (Garau et al., 1978; Kebabian and Calne, 1979) that are now known to be coded by five genes, the products of which are all members of what is termed either the 7 transmembrane (7TM) or G protein-coupled receptor (GPCR) superfamily. The D₂-like family is coded by three different genes yielding D₂, D₃ and D₄ receptors. The other two genes code for the D₁ and D₅ (Dearry et al., 1990; Monsma et al., 1990; Sunahara et al., 1990; Zhou et al., 1990) (Sunahara et al., 1991; Tiberi et al., 1991), the ones of immediate importance to this application. The dopamine receptors have been the subject of numerous reviews and books (Huang et al., 2001; Jenner and Demirdemar, 1997; Neve and Neve, 1997; Neve et al., 2004; Sealfon and Olanow, 2000). For the purposes herein, references to D₁ or D₂ agonist shall refer to actions at the pharmacological subclasses (e.g., D₁ means the D₁-like receptors D₁ and D₅; and D₂ means the D₂-like D₂, D₃, and D₄) unless otherwise specified.

First described in the early 19th century (Parkinson, 1817), PD is the disorder with the clearest link to dopamine dysfunction. The motor effects are due principally to degeneration of the dopaminergic cells in the substantia nigra pars compacta, with consequent loss of dopamine terminals in the striatum. Numerous etiological mechanisms have been

It would be desirable to provide new compounds and methods for treating and preventing conditions such as CNS disorders by administering a dopamine agonist or a dopamine drug that is functionally selective (Urban et al., 2007) to a patient susceptible to, or suffering from, such a condition or disorder. It would be highly beneficial to provide individuals suffering from certain disorders (e.g. CNS diseases) with interruption of the symptoms of those disorders by administering a pharmaceutical composition containing an active ingredient having dopamine pharmacology, and which has a beneficial effect (e.g. upon the functioning of the CNS) but which does not provide any significant associated side-effects. It would be highly desirable to provide a pharmaceutical composition incorporating a compound which interacts with dopamine receptors, such as those which have the potential to affect the functioning of the CNS, but which compound when employed in an amount sufficient to affect the functioning of the CNS, does not significantly affect those receptor subtypes which have the potential to induce undesirable side effects (e.g., emesis and nausea or psychotic-like effects).

WO 96/02513 relates to optically active isomers of dihydrexidine and its substituted analogs.

### SUMMARY OF THE INVENTION

According to a first aspect of the present invention there is provided use of a pharmaceutical composition for the manufacture of a medicament for treating a disorder selected from the group consisting of Parkinson's disease, parkinsonism, restless leg syndrome, schizophrenia, substance abuse, Huntington's chorea, tardive dyskinesia, mood and anxiety disorders, Tourette's syndrome, neurodegeneration resulting from acute events like stroke, and renal, or pulmonary dysfunction, and wherein the composition has a duration of action sufficient for administration no more than three-times daily and that causes typical and/or functionally selective activation of one of more dopamine receptors for oral administration comprising a compound of the formula: 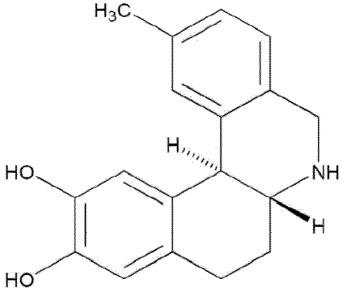 and a pharmaceutically acceptable carrier, wherein the compound is present in an amount of 0.5 to 5 mg/kg. The biological activities of the compounds described herein are known to vary significantly in their selectivity for the dopamine receptor subtypes , depending on the nature and positioning of the substituent groups. Substitution at the C₂, C₃, and/or C₄ position on the benzophenanthridine ring system provides a means for controlling receptor affinity and concomitantly receptor selectivity (Knoerzer et al., 1995). In addition, dihydrexidine has been shown to have unusual functional properties at the D₂ dopamine receptor called "functional selectivity" (Kilts et al., 2002; Mailman, 2007; Mottola et al., 2002; Urban et al., 2007). The ability to utilize these unique properties has been hindered by the extremely short duration of action of these compounds, a limitation overcome by this invention.

The biological activities of the compounds described herein are known to vary significantly in their selectivity for the dopamine receptor subtypes, depending on the nature and positioning of the substituent groups. Substitution at the C2, C3, and/or C4 position on the benzophenanthridine ring system provides a means for controlling receptor affinity and concomitantly receptor selectivity (Knoerzer et al., 1995). In addition, dihydrexidine has been shown to have unusual functional properties at the D2 dopamine receptor called "functional selectivity" (Kilts et al., 2002; Mailman, 2007; Mottola et al., 2002; Urban et al., 2007). The ability to utilize these unique properties has been hindered by the extremely short duration of action of these compounds, a limitation overcome by this invention.

The present compound can be administered, for example, by oral or parenteral routes of administration in amounts effective to evoke therapeutic responses in patients suffering from a variety of disorders which includes Parkinson's Disease, cognitive impairment including that occurring in Alzheimer's disease, schizophrenia, substance abuse, pulmonary function.

The claimed compound is trans-2-methyl-5,6,6a,7,8,12b-hexahydrobenzo[a]phenanthridine, the 2-methyl analog of dihydrexidine herein referred to as Compound 1.

Additional objects, and advantages of the invention, will become apparent to those skilled in the art upon consideration of the following detailed description of preferred embodiments exemplifying the best mode of carrying out the invention.

### BRIEF DESCRIPTION OF THE DRAWINGS

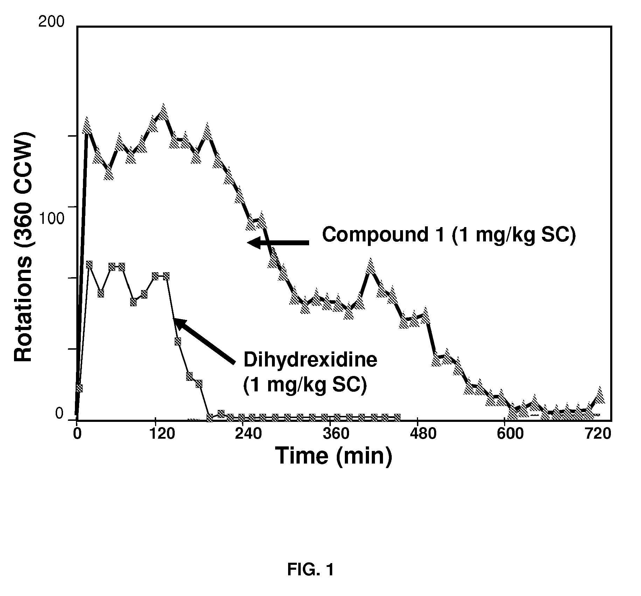

FIG. 1 is a graph showing the duration of activity of Compound 1 as compared to dihydrexidine (DHX) measured is terms of rotations (360 CCW) over time (minutes). Both drugs were administered 1mg/kg subcutaneously (SC), and the number of rotations measured result from inappropriate levels of neurotransmitter release, inappropriate properties of neurotransmitter receptors, and/or inappropriate interaction between neurotransmitters and neurotransmitter receptors, Several CNS disorders involve alterations in dopamine function or can be treated symptomatically or prophylactically with drugs that affect dopamine function. Such CNS disorders include Parkinson's disease, schizophrenia, tardive dyskinesia, Huntington's chorea, anxiety, mood disorder, and Tourette's syndrome among others.

It would be desirable to provide new compounds and methods for treating and preventing conditions such as CNS disorders by administering a dopamine agonist to a patient susceptible to or suffering from such a condition or disorder. It would be highly beneficial to provide individuals suffering from certain disorders (e.g., CNS diseases) with interruption of the symptoms of those disorders by administering a pharmaceutical composition containing an active ingredient having dopamine pharmacology, and which has a beneficial effect (e.g., upon the functioning of the CNS) but which does not provide any significant associated side-effects. It would be highly desirable to provide a pharmaceutical composition incorporating a compound which interacts with dopamine receptors, such as those which have the potential to affect the functioning of the CNS, but which compound when employed in an amount sufficient to affect the functioning of the CNS, does not significantly affect those receptor subtypes which have the potential to induce undesirable effects (e.g., appreciable activity at skeletal muscle and ganglia sites).

### SUMMARY OF THE INVENTION

C₂, C₃, and/or C₄-substituted trans-5,6,6a, 7,8,12b-hexahydrobenzo[a]phenanthridines, oxa-, thio, and azo substituted analogs thereof, compositions including these compounds, and methods of treatment using the compounds, are disclosed. In one embodiment, the compounds include common structural features with those compounds described in U.S. Patent Nos. 5,047,536 and 5,420,134. They are particularly related to the compound trans-10,11-dihydroxy-5,6,6a,7,8,12b-hexahydrobenzo-[a]phenanthridine (dihydrexidine), a D₁ agonist which has been the subject of much study over the past several years, except that the compounds described herein have an enhanced duration of action relative to the compounds in the '536 and '134 patents.
The compounds described herein are 2-substituted analogs of the dihydrexidine series. The compounds in the '536 and '134 patents are, in some cases, full D₁ dopamine agonists, and in other cases, analogs with varying degrees of D₁:D₂ binding affinity, with the ability to differentially activate functions mediated by the dopamine D₂ receptor. All of the reported examples, however, were limited as potential drugs by extremely short durations of action. By adding small substituents to the 2-position, the compounds described herein have marked and unexpected increases in duration of action without markedly altering the receptor profile of the parent compound. While not wishing to be bound to a particular theory, it is believed that this is due to intrinsic steric protection against the conjugating activity of enzymes directed at the catechol moiety, and is like adding a metabolism inhibitor to the drug.

The biological activities of the compounds described herein are known to vary significantly in their selectivity for the dopamine receptor subtypes, depending on the nature and positioning of the substituent groups. Substitution at the C₂, C₃, and/or C₄ position on the benzophenanthridine ring system provides a means for controlling receptor affinity and concomitantly receptor selectivity (Knoerzer et al., 1995). In addition, dihydrexidine has been shown to have unusual functional properties at the D₂ dopamine receptor called "functional selectivity" ( Mottola et al., 2002; Kilts et al., 2002; Urban et al., 2007; Mailman, 2007;). The ability to utilize these unique properties has been hindered by the extremely short duration of action of these compounds, a limitation overcome by this invention.

The present compound can be administered, for example, by oral or parenteral routes of administration in amounts effective to evoke therapeutic responses in patients suffering from Parkinson's disease and schizophrenia.

The compound is trans-2-methyl-5,6,6a, 7,8,12b-hexahydrobenzo[a]phenanthridine, the 2-methyl analog of dihydrexidine herein referred to as Compound 1.

Additional objects, and advantages of the invention, will become apparent to those skilled in the art upon consideration of the following detailed description of preferred embodiments exemplifying the best mode of carrying out the invention.

### BRIEF DESCRIPTION OF THE DRAWINGS

FIG. 1 is a graph showing the duration of activity of Compound 1 as compared to dihydrexidrine (DHX) measured in terms of rotations (360 CCW) over time (minutes). Both drugs were administered 1 mg/kg subcutaneously (SC), and the number of rotations measured (i.e., rotations counter clockwise) over time (minutes) using the model first described by Ungerstedt (Ungerstedt and Arbuthnott, 1970).

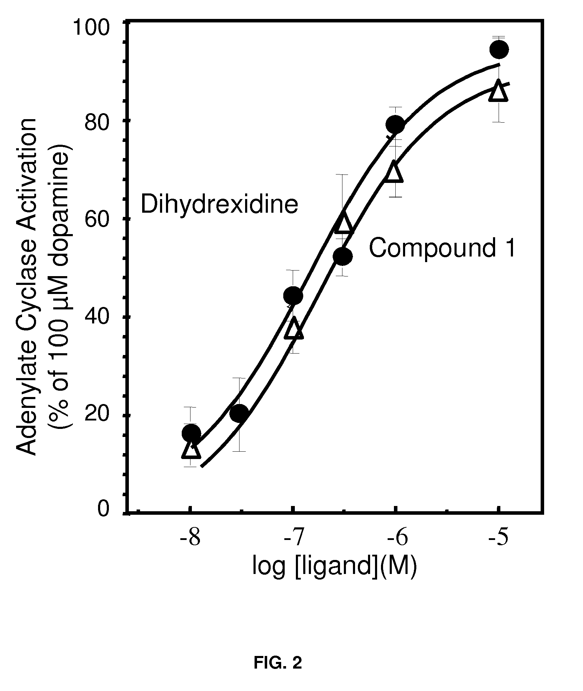

FIG. 2 is a graph showing that Compound 1 is a full dopamine D₁ receptor agonist. In Fig. 2, Compound 1 is compared to dihydrexidine in their ability to activate adenylate cyclase (shown as a percentage of activation caused by 100 µM dopamine vs. the log concentration for Compound 1 or dihydrexidine).

### DETAILED DESCRIPTION

The present invention relates to the use of 2-methyl dihydrexidine as D₁ agonists, or which have activity at both the D₁ and D₂ receptor subtypes, as well as compositions including the compounds, and methods of treatment using the compounds.

### Definitions:

The term "C₁ -C₄ alkyl" as used herein refers to branched or straight chain alkyl groups comprising one to four carbon atoms, including, but not limited to, methyl, ethyl, propyl, isopropyl, n-butyl, t-butyl and cyclopropylmethyl.

The term "pharmaceutically acceptable salts" refers to those salts which are suitable for use in contact with the tissues of humans and lower animals without undue toxicity, irritation, allergic response and the like, and are commensurate with a reasonable benefit/risk ratio. Pharmaceutically acceptable salts are well known in the art. The salts can be prepared according to conventional methods in situ during the final isolation and purification of the compounds, or separately by reacting the free base with a suitable organic acid.
The term "phenoxy protecting group" as used herein refers to substituents on the phenolic oxygen which prevent undesired reactions and degradations during synthesis and which can be removed later without effect on other functional groups on the molecule. Such protecting groups and the methods for their application and removal are well known in the art. They include ethers, such as methyl, isopropyl, t-butyl, cyclopropylmethyl, cyclohexyl, allyl ethers and the like; alkoxyalkyl ethers such as methoxymethyl or methoxyethoxymethyl ethers and the like; alkylthioalkyl ethers such a ligands with unexpected pharmacokinetic and pharmacodynamic properties. Ideally, the duration of action is sufficient for administration no more than three times daily. Typically, the compounds cause typical or functionally-selective activation of one of more dopamine receptors.

The differences between the claimed compounds, and those described in the prior art, can be more clearly seen with reference to the following structures. As shown below, the compound Compound 1 differs from dihydrexidine by the presence of a methyl group in position R₁.

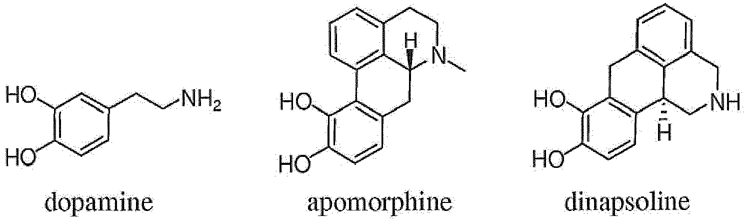

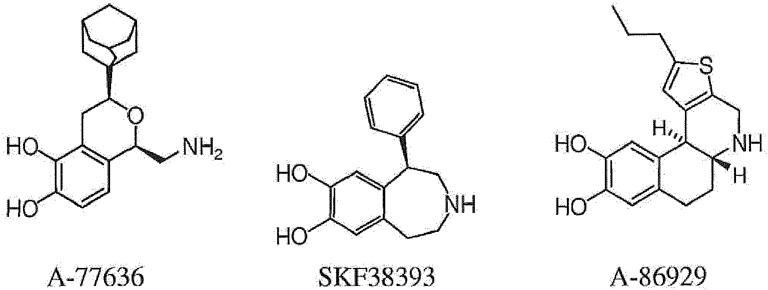

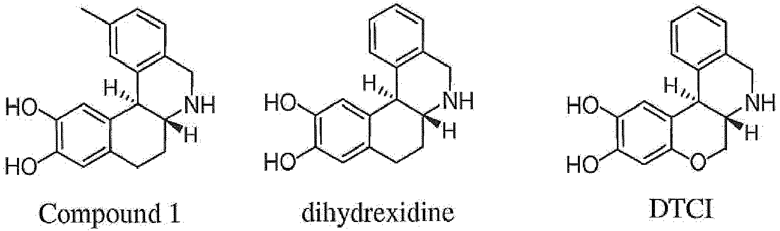

### Structures of Compound 1 and other prototypical D₁ agonists.

### II. Methods of Preparing the Compounds

The compounds of this invention are prepared using the same preparative chemical steps described for the preparation of the hexahydrobenzo[a]phenanthridine compounds described and claimed in U.S. Pat. No. 5,047,536, issued Sep. 10, 1991. The present compounds can be prepared using the chemical dopamine. As used herein, an "antagonist" is a substance that binds to its target receptor, in this case, the dopamine D₁ receptor, or the dopamine D₁ and D₂ receptors, and causes no effect by itself but blocks the actions of dopamine or a dopamine agonist that might be present. As used herein, a "partial agonist" is a substance that binds to its target receptor, and no matter how high the concentration, causes a functional effect that is intermediate between that caused by a full agonist and an antagonist. The term "intrinsic activity" or "efficacy" as used herein relates to the measure of biological action caused in a particular assay system. In some circumstances, intrinsic activity may vary depending on the particular second messenger system involved (see Hoyer and Boddeke, 1993) (Urban et al., 2007; Mailman, 2007). In addition, some drugs can have markedly different intrinsic activity when one measures different signaling pathways mediated by the same receptor. This phenomenon is most commonly termed "functional selectivity" ( Mailman and Gay, 2004; Mailman and Huang, 2007; Urban et al., 2007; Mailman 2007). Where such contextually specific evaluations are relevant, and how they might be relevant in the context of the present invention, will be apparent to one of ordinary skill in the art.

### I. Compounds

The compounds described herein include C₂, C₃, and/or C₄ -substituted trans-5,6,6a, 7,8,12b-hexahydrobenzo[a]phenanthridines, oxa-, thio, and azo substituted analogs thereof, prodrugs or metabolites of these compounds, and pharmaceutically acceptable salts thereof. The compounds can bind to, and either specifically modulate dopamine D₁ receptors, or modulate both dopamine D₁ and D₂ receptors, in the patient's brain in what are termed mesocortical, mesolimbic, nigrostriatal, and tuberoinfundibulkar terminal fields. When so bound, the compounds may affect the signaling of dopamine receptors in both pre- and postsynaptic cells.

Receptor binding constants are a quantitative measure of the ability of a compound to bind to its target receptor(s). See, for example, Cheng and Prusoff (1973). The receptor binding constants of the compounds described herein generally exceed about 0.1 nM, often exceed about 1 nM, and frequently exceed about 10 nM, and are often less than about 100 µm, often less than about 10 µm and frequently less than about 5 µm. Preferred compounds generally have receptor binding constants for the D₁ receptor less than about 1 µM, and can be less than about 100 nM.

Preferably, the compounds can cross the blood-brain barrier, and thus enter the central nervous system of the patient. Log P values provide a measure of the ability of a compound to pass across a diffusion barrier, such as a biological membrane, including the blood brain barrier (Hansch et al., 1995). Typical log P values for the compounds described herein are generally greater than about -0.5, often are greater than about 0, and frequently are greater than about 0.5, and are typically less than about 3, often are less than about 2, and frequently are less than about 1.

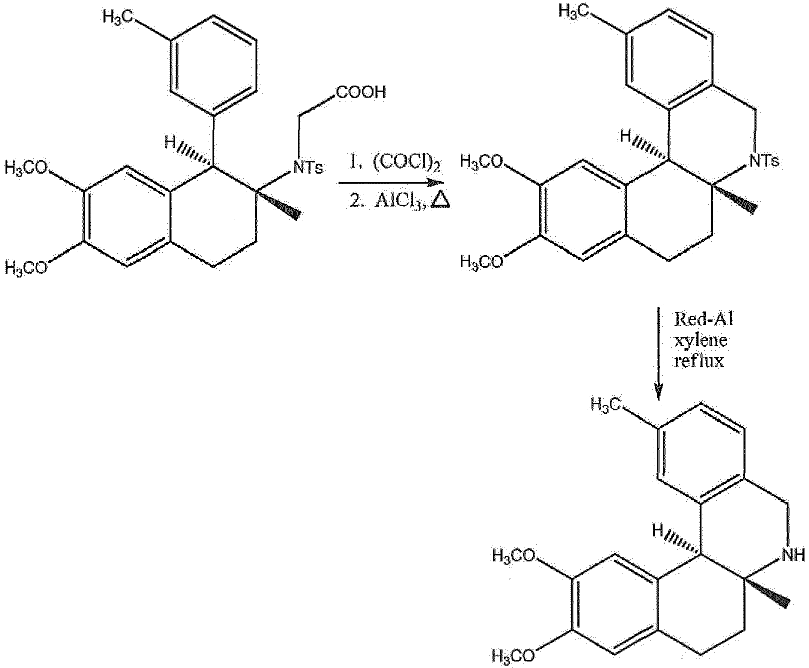

### III. Pharmaceutical Compositions

The compounds described herein can be formulated in conventional drug dosage forms. Preferred doses of the present compounds depend on many factors, including the indication being treated, the route of administration, and the overall condition of the patient. For oral administration, for example, effective doses of the present compounds are expected to range from 0.1 to 25 mg/kg, more typically 0.5 to 5 mg/kg. Effective parenteral doses can range from 0.01 to 5 mg/kg of body weight, more typically from 0.1 to 1 mg/kg of body weight. In general, treatment regimens utilizing compounds in accordance with the present invention comprise administration of from 1 mg to 500 mg of the compounds of this invention per day in multiple doses or in a single dose.

### Liquid Dosage Forms

Liquid dosage forms for oral administration may include pharmaceutically acceptable emulsions, microemulsions, solutions, suspensions, and syrups containing inert diluents commonly used in the art, such as water. Such compositions may also comprise adjuvants such as wetting agents, emulsifying and suspending agents, sweetening, and flavoring agents. Injectable preparations of the compounds of the present invention can be formulated utilizing art-recognized procedures by dispersing or dissolving an effective dose of the compound in a parenterally acceptable diluent such as water, or more preferably isotonic sodium chloride solution. The parenteral formulations can be sterilized using art-recognized microfiltration techniques.

### Solid Dosage Forms

The compounds of this invention can also be formulated as solid dosage forms for oral administration such as capsules, tablets, powders, pills and the like. Typically the active compound is admixed with an inert diluent or carrier such as sugar or starch and other excipients appropriate for the dosage form. Thus, tablet formulations will include acceptable lubricants, binders and/or disintegrants. Optionally powder compositions comprising an active compound of this invention and, for example, a starch or sugar carrier can be filled into gelatin capsules for oral administration.

Other dosage forms of the compounds of the present invention can be formulated using art-recognized techniques in forms adapted for the specific mode of administration. This can include novel formulations such as Zydis, which is an oral disintegrating tablet including gelatin, mannitol, sodium methyl paraben, and sodium propyl paraben, and other oral disintegrating tablets.

Nanoparticulate delivery systems can also be used. Nanoparticulate compositions, first described in U.S. Pat. No. 5,145,684, include particles comprising a therapeutic agent having adsorbed onto the surface thereof a non-crosslinked surface stabilizer.

Methods of making nanoparticulate compositions are described, for example, in U.S. Pat. Nos. 5,518,187 and 5,862,999, both for "Method of Grinding Pharmaceutical Substances;" U.S. Pat. No. 5,718,388, for "Continuous Method of Grinding Pharmaceutical Substances;" and U.S. Pat. No. 5,510,118 for "Process of Preparing Therapeutic Compositions Containing Nanoparticles."

Nanoparticulate compositions are also described, for example, in U.S. Pat. No. 5,298,262 for "Use of Ionic Cloud Point Modifiers to Prevent Particle Aggregation During Sterilization;" U.S. Pat. No. 5,302,401 for "Method to Reduce Particle Size Growth During Lyophilization;" U.S. Pat. No. 5,318,767 for "X-Ray Contrast Compositions Useful in Medical Imaging;" U.S. Pat. No. 5,326,552 for "Novel Formulation For Nanoparticulate X-Ray Blood Pool Contrast Agents Using High Molecular Weight Non-ionic Surfactants;" U.S.

Optically active forms of the compound can be prepared using any method known in the art, including but not limited to by resolution of the racemic form by recrystallization techniques, by synthesis from optically-active starting materials, by chiral synthesis, or by chromatographic separation using a chiral stationary phase.

Space-filling representations of the low energy conformations for (+)-trans-10,11-dihydroxy-5,6,6a,7,8,12b-hexahydrobenzo[a]phenanthridine [(+)-dihydrexidine] and a variety of other high intrinsic activity full D₁ agonists have been compared (Mottola et al., 1996). Based on the underlying model of the D₁ pharmacophore, it is anticipated that both the affinity and intrinsic activity of racemic Compound 1 (and substituted analogs thereof, as described herein) reside in only one of its enantiomers - the 6aR, 12bS absolute configuration (and its homochiral analogs). Resolution of the racemate using art recognized separation techniques is expected to yield one Compound 1 isomer with approximately twice the D₁ affinity exhibited by racemic Compound 1, thus making its affinity for the D₁ receptor similar to (+)-dihydrexidine. It also has been shown that the D₂ properties of dihydrexidine reside in the same enantiomer (i.e., 6aR, 12bS) that is the high affinity full agonist at the D₁ receptor. On this basis, it is expected that both the D₁ and D₂ properties of Compound 1 will also reside in the homochiral enantiomer. Thus, in addition to the importance of the actions at D₁ receptors, the optical isomers of Compound 1 and appropriate analogs may constitute significant tools to study the phenomena of "functional selectivity" (Urban et al., 2007; Mailman, 2007;).

Compounds, pharmaceutical compositions including the compounds, and methods of treatment, in which the compounds are enantiomerically-enriched in the 6aR, 12bS enantiomer, are also described herein but not within the scope of the invention.

The compounds are dopamine receptor ligands with unexpectedly longer duration of action than the parent ligands of this class. The longer duration of action caused by the 2-position substituents can be used in concert with substitution at other positions to create novel ligands with unexpected pharmacokinetic and pharmacodynamic properties. Ideally, the duration of action is sufficient for administration no more than three times daily. Typically, the compounds cause typical or functionally-selective activation of one of more dopamine receptors.

The differences between the compounds described herein, and those described in the prior art, can be more clearly seen with reference to the following structures. As shown below, the compound Compound 1 differs from dihydrexidine by the presence of a methyl group in position R₁.

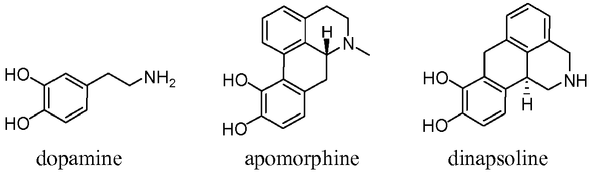

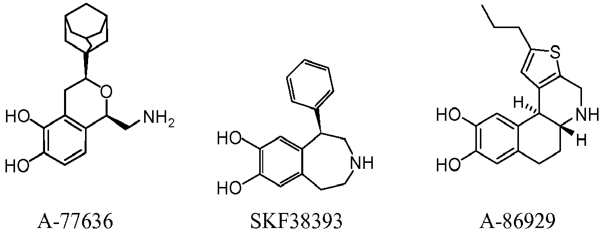

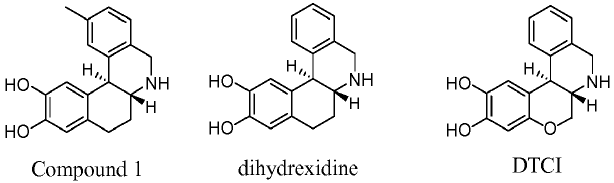

### Structures of Compound 1 and other prototypical D₁ agonists.

### II. Methods of Preparing the Compounds

The compounds described herein are prepared using the same preparative chemical steps described for the preparation of the hexahydrobenzo[a]phenanthridine compounds described and claimed in U.S. Pat. No. 5,047,536, issued Sep. 10, 1991. The present compounds can be prepared using the chemical reactions depicted in the reaction scheme illustrated in FIGS. 1 and 2 of U.S. Pat. No. 5,047,536 using the appropriately substituted benzoic acid acylating agent starting material instead of the benzoyl chloride reagent used in the initial reaction step. Thus, for example, use of 4-methylbenzoyl chloride will yield a 2-methyl hexahydrobenzo[a]phenanthridine compound.

Embodiments of general reaction schemes are shown below as Scheme I.

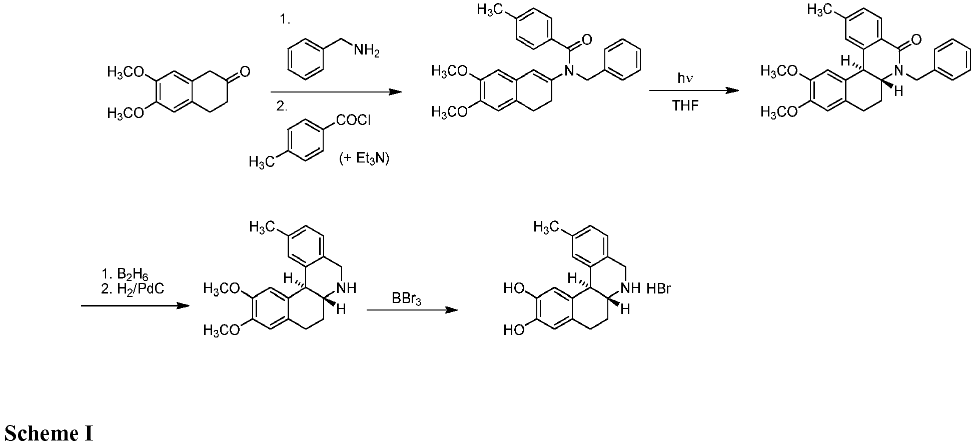

As shown in Scheme I, a 6,7,-difunctionalized-beta-tetralone is reacted with benzylamine in a suitable solvent, such as toluene, to provide the benzyl imine analogue. To arrive at the compounds described herein, the tetralone is suitably functionalized at

Certain pharmaceutically acceptable salts tend to be more preferred for use in transdermal formulations, because they can help the active substance pass the barrier of the stratum corneum. Examples include fatty acid salts, such as stearic acid and oleic acid salts. Oleate and stearate salts are relatively lipophilic, and can even act as a permeation enhancer in the skin.

Permeation enhancers can also be used. Representative permeation enhancers include fatty alcohols, fatty acids, fatty acid esters, fatty acid amides, glycerol or its fatty acid esters, N-methylpyrrolidone, terpenes such as limonene, alpha-pinene, alpha-terpineol, carvone, carveol, limonene oxide, pinene oxide, and 1,8-eucalyptol.

The patches can generally be prepared by dissolving or suspending the active agent in ethanol or in another suitable organic solvent, then adding the adhesive solution with stirring. Additional auxiliary substances can be added either to the adhesive solution, the active substance solution or to the active substance-containing adhesive solution. The solution can then be coated onto a suitable sheet, the solvents removed, a backing layer laminated onto the matrix layer, and patches punched out of the total laminate.

### Inhalable Formulations

In one embodiment, the compounds are administered via inhalable (i.e., pulmonary and intranasal) formulations. These can be particularly useful for treating patients suffering from Parkinson's Disease, where later stage patients have trouble swallowing.

In another embodiment, the present invention relates to a method of administering the compounds described herein to a patient, comprising administering to the patient a therapeutically effective amount of the pulmonary or intranasal compositions described herein.

### Intranasal Formulations

In one embodiment, the present invention relates to an intranasal pharmaceutical formulation containing a pharmaceutically acceptable salt of the compounds described herein. Such intranasal formulations are useful for treating disorders where the administration of the compounds is beneficial, in particular in the treatment of Parkinson's Disease, and with patients who have difficulty swallowing orally-administered formulations.

Ideally, the formulations have no detectable microbiological contamination. In one embodiment, this is achieved without using potentially irritating preservatives, such as ethanol or benzalkonium chloride, even though such preservatives can be added as needed. Ideally, the formulations produce low irritation of the nasal mucosa, and avoid nasal vestibulitis, when administered.

In one aspect of this embodiment, the liquid intranasal pharmaceutical formulation includes a pharmaceutically-acceptable acid addition salt of the compounds described herein, and a cyclodextrine, such as alpha-or beta-cyclodextrin, or methylated versions thereof. Representative pharmaceutically acceptable acid addition salts are described herein, and specifically include hydrochloride, citrate and methanesulfonate.

The liquid intranasal formulation can further include buffer salts, e.g. phosphates or acetates, and as such may be present as a buffered aqueous solution, for example, phosphate buffered saline (PBS).

The intranasal formulation can also further include a viscosity-enhancing substance. Glycerol and carboxymethylcellulose (CMC) are non-limiting examples of such viscosity enhancers. Glycerol can be particularly preferred, as it also has a soothing effect on the nasal mucosa.

In one aspect of this embodiment, the viscosity of the intranasal formulations is between 0.8 and 1.5 mm²/s, for example, around 1.2 mm²/s. The viscosity can be determined, for example, by an Ubbelohde capillary viscosimeter with suspending ball-level for the determination of kinematic viscosity according to DIN 51562, part 1.

The pH-value of the intranasal formulations is ideally in the range of 4.5 to 6.5, more preferably around 5.8 +/- 0.3. This pH range can provide an optimum between good drug stability and solubility, and good flux across the nasal mucosal membrane (which tends to be better at around pH 7). The pH value of the intranasal formulation can be adjusted during or after its preparation with a pharmaceutically acceptable acid or base, for example, citric acid or a citrate salt.

In one aspect, the intranasal formulation does not contain a further absorption enhancer, preservative and/or antioxidant. In other aspects, the intranasal formulations contain further absorption enhancers. Such enhancers include, but are not limited to, surfactants and/or emulsifiers, particularly non-ionic surfactants such as TWEEN 80 TM or cremophor RH40™. Representative antioxidants include ascorbates or sorbates. Representative preservatives include, but are not limited to, antimicrobial substances such benzalkonium chloride.

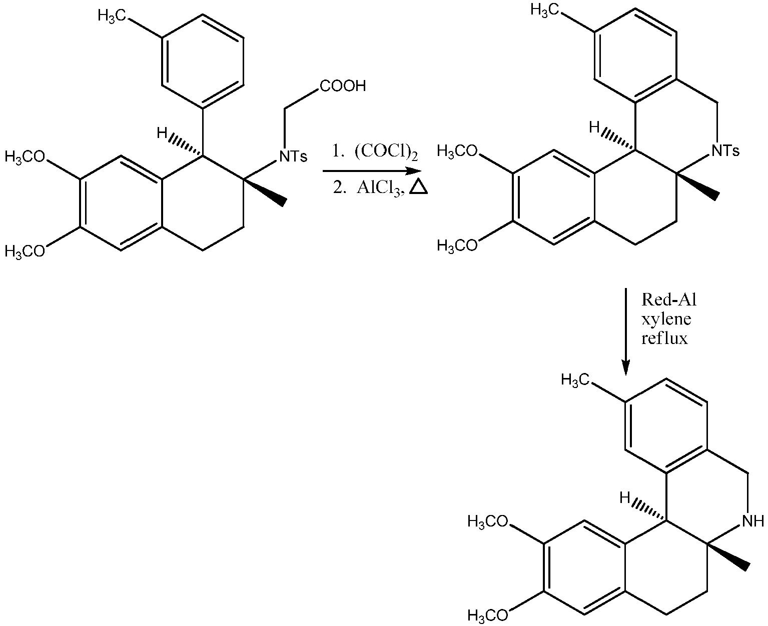

### III. Pharmaceutical Compositions

The compounds described herein can be formulated in conventional drug dosage forms. Preferred doses of the present compounds depend on many factors, including the indication being treated, the route of administration, and the overall condition of the patient. For oral administration, for example, effective doses of the present compounds range from about 0.5 to 5 mg/kg.

### Liquid Dosage Forms

Liquid dosage forms for oral administration may include pharmaceutically acceptable emulsions, microemulsions, solutions, suspensions, and syrups containing inert diluents commonly used in the art, such as water. Such compositions may also comprise adjuvants such as wetting agents, emulsifying and suspending agents, sweetening, and flavoring agents. Injectable preparations of the compounds of the present invention can be formulated utilizing art-recognized procedures by dispersing or dissolving an effective dose of the compound in a parenterally acceptable diluent such as water, or more preferably isotonic sodium chloride solution. The parenteral formulations can be sterilized using art-recognized microfiltration techniques.

### Solid Dosage Forms

The compound of this invention can also be formulated as solid dosage forms for oral administration such as capsules, tablets, powders, pills and the like. Typically the active compound is admixed with an inert diluent or carrier such as sugar or starch and other excipients appropriate for the dosage form. Thus, tablet formulations will include acceptable lubricants, binders and/or disintegrants. Optionally powder compositions comprising an active compound of this invention and, for example, a starch or sugar carrier can be filled into gelatin capsules for oral administration.

Other dosage forms of the compound of the present invention can be formulated using art-recognized techniques in forms adapted for the specific mode of administration.

through the airways can be prepared in aqueous or non-aqueous vehicles, and delivered to the airways by means of drops or sprays.

Thus, in one embodiment, the present invention relates to a composition for pulmonary delivery comprising a compound described herein dispersed in an aqueous or non-aqueous delivery vehicle. The aqueous vehicle is selected from pure water, substantially pure water or water combined with other excipients such as salts, ions or other excipients which are generally used in aqueous based systems. The liquid formulations are in the form of solution based dispersions or solutions in solvents or cosolvents such as alcohols or glycols with water. Non-aqueous solutions include those alchohol or glycol based systems which may have some water, but which are not comprised of a majority percentage of water and which are known to those of skill in the art as effective and safe delivery vehicles. Non-aqueous solutions also include those systems containing halogenated hydrocarbons. Administration of liquid formulations in the form of drops or dispersions occurs through the nose and/or trachea to facilitate absorption of the formulation and prodrug and/or active ingredients into the lungs and ultimately delivery to the dopamine receptors where the medicinal effect is achieved to treat, for example, Parkinson's disease. Devices can be used to assist in the delivery of the active agent(s).

In another embodiment, the present invention provides a pharmaceutical composition for aerosol delivery of a compound described herein, which include, in addition to the active agent, a propellant, poloxamer and tocopherol.

Numerous chlorofluorocarbon (CFC) and non-chlorofluorocarbon (NCFC) aerosol propellants can be used. Representative CFC propellants include CFC-11 (trifluorochloromethane), CFC-12 (dichlorodifluoromethane) and CFC-114 (dichlorotetrafluoroethane). Ideally, the propellants are non-ozone depleting halogenated alkanes such as HCFC-123 (1,1,1-trifluoro-2,2-dichloroethane), HCFC-124 (1,1,1,2-tetrafluorochloroethane), HCFC-141b, HCFC-225, HFC-125, FC-C51-12 (perfluorodimethylcyclobutane), DYMEL A (dimethyl ether), DYMEL 152a (1,1-difluoroethane), HFC-134a, and HFC-27ea.

The poloxamers that can be used in the compositions are block copolymers of ethylene oxide and propylene oxide. The poloxamers typically have a molecular weight of from about 1950 to about 3350 and a hydrophilic lipophilic balance (hlb) of from about 10 to about 20. Representative poloxamers include poloxamer 124 (Pluronic.RTM. L44, MW about 2200, hlb 16), Pluronic.RTM. 10R5 (MW about 1950, hlb 15), Pluronic.RTM. 17R4 (MW about 2650, hlb 12), Pluronic.RTM. 22R4 (MW about 3350, hlb 10) and Pluronic.RTM. L64 (MW about 2900, hlb 15), all available from BASF Corp., Parsippany, N.J.

Poloxamer can be present in a concentration of from about 0.001% to about 5%, preferably in a concentration of from about 0.01% to about 2% and most preferably in a concentration of from about 0.1% to about 1%. Preferred poloxamers have a molecular weight of from about 1950 to about 2900 and an hlb of from about 12 to about 16. The most preferred poloxamer of the present invention is poloxamer 124.

Poloxamer 124 has the chemical name .alpha.-hydro-.omega.hydroxypoly(oxyethylene)poly(oxypropylene)poly(oxyeth ylene) block copolymer. As listed in USPNF XVII, poloxamer 124 has a molecular weight of between 2090 and 2360 and a hlb of 16. It is a liquid at ambient temperature and has weight percent oxyethylene of 46.7%.+-.1.9% and unsaturation (mEq/g) of 0.020.+-.0.008 see Wade, A. and Weller P. L., eds., Handbook of Pharmacetucial Excipients, (2 ed., Washington, D.C.: American Pharmaceutical Assoc.) 1994, 352-354. Pluronic.RTM. L44 has a molecular weight of about 2250.

The aerosol compositions can also contain additional inactive excipients such as antioxidants and flavoring and/or taste masking agents to stabilize the drug and improve dosimetry. Preferred antioxidants are tocopherol derivatives such as d-alpha tocopherol, dl-alpha tocopherol, d-alpha tocopherol acetate, dl-alpha tocopherol acetate d-alpha tocopherol acid succinate and dl-alpha tocopherol acid succinate. The most preferred antioxidant is dl-alpha tocopherol acetate. The antioxidant may be present in a concentration of from about 0.001% to about 5%, preferably in a concentration of from about 0.01% to about 2% and most preferably in a concentration of from about 0.01% to about 1%.

A sweetener such as aspartame and/or a taste masking agent such as menthol may also be present in concentrations of between about 0.001% and and about 10% by weight, preferably in a concentration of between about 0.002% and about 5% by weight and more preferably in a concentration of between about 0.01% and 1%.

The MDI compositions can be prepared by combining poloxamer and any other excipients with a medicament which has been milled or otherwise reduced to a desired particle size, and placing the mixture in a suitable aerosol container or vial. After sealing the container, an aerosol propellant is introduced and the system is agitated to fully blend the ingredients. In some instances, it may be necessary to wet-mill the medicament in a closed system, as for example under temperature and pressure conditions which permit the medicament to be milled while mixed with a liquid-phase aerosol propellant. It is expected that, for any particular combination of medicament, propellant and poloxamer, the ideal order of addition of ingredients and the conditions under which they are to be combined may readily be determined.

### Uniformity of MDI Delivery

Delivery uniformity of the MDI compositions can be tested, for example, as follows: An aerosol container can be shaken and it valve primed by aerosolizing 5 times in succession. After priming, the aerosol container can be shaken and then attached to an atomizing nozzle which can be cut from an actuator. With the nozzle pointed downward, the canister can be placed into a 30-mL beaker containing 10 mL of methanol until the nozzle touches the bottom of the beaker. Then, a total of 2 sprays, each separated by a 5 second pause, can be delivered into the beaker. The valve stem and ferrule can be rinsed with acetonitrile. The amount of drug in each sample can be analyzed, for example, by HPLC.

### Aerosol Particle Size Distribution

Particle size data in an aerosol formulation can be determined, for example, using the Malvern laser diffraction particle sizer (Model 2600C). Samples can be analyzed as aerosolized aprays in air. An aerosol can with an actuator assembly can be mounted on a clamp stand so that the spray jet is around 12.5 cm from the laser beam. Beam length, i.e., the length of aerosol flume along the path of the laser bean, can be about 10 cm. In this configuration, the distance of the objective lens can be 3 cm from the middle of the aerosol flume, and the IR beam of the spray synthronizer can be 4 cm from the spray jet. Also, the laser beam and the IR beam can be parallel and approximately 8.5 cm apart. A representative number of sprays can be actuated and analyzed individually assuming a log-normal distribution model. The detection of spray duration is approximately 15 milliseconds (ms), i.e., beginning from 70 ms and ending at 85 ms after interruption of the IR beam by the aerosol.

The particle size of aerosolized product determines the extent as well as the pattern of drug deposition in the respiratory tract. Ideally, the emitted particle size is less than 10 microns, preferably less than around 5 microns.

### Bioavailability of MDI Compositions

The bioavailability of the formulations can be assessed, for example, using a non-crossover bioavailability study involving a suspension aerosol formulation and an iv injection solution. The aerosol sprays can be delivered anteriorly via a tracheal stoma in a test animal. Plasma concentration profiles of the active metabolite of the compounds following administration of the formulations can then be compared, and ideally demonstrate that lung absorption of the compound following inhalation delivery occurs at least as efficiently as I.V. administration, or, at a minimum, at sufficient levels to achieve a desired physiological effect.

### Optional Additional Components

The pharmaceutical composition also can include various other components as additives or adjuncts. Exemplary pharmaceutically acceptable components or adjuncts which are employed in relevant circumstances include antioxidants, free radical scavenging agents, other centrally acting drugs, peptides, growth factors, antibiotics, bacteriostatic agents, immunosuppressives, anticoagulants, buffering agents, antiinflammatory agents, antipyretics, time release binders, anesthetics, steroids and corticosteroids. Such components can provide additional therapeutic benefit, act to affect the therapeutic action of the pharmaceutical composition, or act towards preventing any potential side effects which may be posed as a result of administration of the pharmaceutical composition. In certain circumstances, a compound of the present invention can be employed as part of a pharmaceutical composition with other compounds intended to prevent or treat a particular disorder.

### IV. Methods of Treatment

The compounds described herein can be used to treat or prevent a variety of disorders mediated by dopamine neurons or dopamine-target neurons. The methods involve administering to a patient an amount of a compound effective for providing some degree of prevention of the progression, amelioration of the symptoms, or amelioration of the reoccurrence, of a disorder mediated by dopamine systems, for example, neurologic or psychiatric brain disorders.

The compounds described herein are useful for treating those types of conditions and disorders for which other types of dopamine agonists have been proposed as therapeutics. Representative CNS disorders that can be treated include Parkinson's disease, parkinsonism, restless leg syndrome, schizophrenia, presenile dementia (early onset Alzheimer's disease), senile dementia (dementia of the Alzheimer's type), and other disorders with cognitive deficits (including age-associated cognitive deficits), substance abuse, tardive dyskinesia, addition, U.S. patent application No. 20020012675 A1, published on Jan. 31, 2002, for "Controlled Release Nanoparticulate Compositions," describes nanoparticulate compositions.

### Transdermal Formulations

The claimed composition can be present in the form of transdermal formulations, such as that used in the FDA-approved agonist rotigitine transdermal (Neupro patch). Another suitable formulation is that described in U.S. Publication No. 20080050424, entitled "Transdermal Therapeutic System for Treating Parkinsonism." This formulation includes a silicone or acrylate-based adhesive, and can include an additive having increased solubility for the active substance, in an amount effective to increase dissolving capacity of the matrix for the active substance.

The transdermal formulations can be single-phase matrices that include a backing layer, an active substance-containing self-adhesive matrix, and a protective film to be removed prior to use. More complicated embodiments contain multiple-layer matrices that may also contain non-adhesive layers and control membranes. If a polyacrylate adhesive is used, it can be crosslinked with multivalent metal ions such as zinc, calcium, aluminum, or titanium ions, such as aluminum acetylacetonate and titanium acetylacetonate.
When silicone adhesives are used, they are typically polydimethylsiloxanes. However, other organic residues such as, for example, ethyl groups or phenyl groups may in principle be present instead of the methyl groups. Because the active compounds are to an undesirable level, as is demonstrated by decreased effects on preparations believed to reflect effects on the cardiovascular system. As such, administration of compounds of the present invention provides a therapeutic window in which treatment of certain CNS disorders is provided, and side effects are minimal. That is, an effective dose of the compounds described herein are sufficient to provide the desired effects upon the CNS, but is insufficient (i.e., is not at a high enough level) to provide undesirable side effects at a level that would eliminate the utility of these compounds. Preferably, effective administration of a compound resulting in treatment of CNS disorders occurs upon administration of less than one-half, frequently less than one-fifth, and often less than one-tenth, that amount sufficient to cause side effects that would prevent a compound's clinical use.

### Treatment of Parkinson's Disease

The efficacy of selective or non-selective D₂ agonists has largely been disappointing, especially in late-stage, severely disabled PD patients. Dopamine agonists are effective in delaying levodopa-induced dyskinesia in early PD and reducing motor fluctuations in advanced PD, but a recent commentary as levodopa approached its fortieth birthday said "For the foreseeable future, levodopa will remain the gold standard for Parkinson's treatment" (Haughn, 2007). Table 1 summarizes the properties of the available dopamine agonists (noting that many of these are coming off-of-the-market because of cardiac valvuopathy problems). There has been some controversy about the relative importance of the D₂ and D₃ dopamine receptor isoforms as regards the antiparkinson actions of pramipexole and ropinirole. A variety of evidence seems to suggest that it is the D₂, not D₃, properties of these drugs that drives their antiparkinson action (Mailman and Huang, 2007), but except for apomorphine *(vide infra*), no FDA-approved dopamine agonist comes close to matching a levodopa/adjuvant combination for antiparkinson efficacy.

Currently there are several drugs approved for human use for Parkinson's disease. In the United States this includes pramipexole, ropinirole, rotigitine, and apomorphine. Other ergoline-based molecules (such as pergolide) have either been removed from the market or "black-boxed" because they induce cardiac valve pathology. All of the FDA-approved compounds have highest affinity for D₂ dopamine receptors, and with one exception, they all have but modest efficacy in Parkinson's disease. The exception is apomorphine that is effective as monotherapy, and is the only one of these approved drugs that has high D₁ intrinsic activity. Unfortunately, apomorphine, like dihydrexidine, has a very short duration of action. In addition, its D₂ properties decrease its tolerability (e.g., nausea and emesis).

the active substance solution or to the active substance-containing adhesive solution. The solution can then be coated onto a suitable sheet, the solvents removed, a backing layer laminated onto the matrix layer, and patches punched out of the total laminate.

### Optional Additional Components

The pharmaceutical composition also can include various other components as additives or adjuncts. Exemplary pharmaceutically acceptable components or adjuncts which are employed in relevant circumstances include antioxidants, free radical scavenging agents, other centrally acting drugs, peptides, growth factors, antibiotics, bacteriostatic agents, immunosuppressives, anticoagulants, buffering agents, anti-inflammatory agents, antipyretics, time release binders, anesthetics, steroids and corticosteroids. Such components can provide additional therapeutic benefit, act to affect the therapeutic action of the pharmaceutical composition, or act towards preventing any potential side effects which may be posed as a result of administration of the pharmaceutical composition. In certain circumstances, a compound of the present invention can be employed as part of a pharmaceutical composition with other compounds intended to prevent or treat a particular disorder.

### IV. Methods of Treatment

The compound described herein can be used to treat or prevent a variety of disorders mediated by dopamine neurons or dopamine-target neurons. The methods involve administering to a patient an amount of a compound effective for providing some degree of prevention of the progression, amelioration of the symptoms, or amelioration of the reoccurrence, of a disorder mediated by dopamine systems, for example, neurologic or psychiatric brain disorders.

The compound described herein are useful for treating those types of conditions and disorders for which other types of dopamine agonists have been proposed as therapeutics. Representative CNS disorders that can be treated include Parkinson's disease, parkinsonism, restless leg syndrome, schizophrenia, Huntington's chorea, Tourette's syndrome, and neurodegeneration resulting from acute events like stroke, renal dysfunction, and lung conditions.

In addition, the compound can be used to improve the cognitive function of "normal" patients, i.e., those who do not manifest clinical signs of cognitive deficit. Thus, for example, "normal" individuals using the compounds described herein, may evidence improvement in memory, cognition, and/or concentration.

The compound has the ability to pass across the blood-brain barrier of the patient. As such, such compound has the ability to enter the central nervous system of the patient. The log P values of typical compounds, which are useful in carrying out the present invention are generally greater than about 0, often are greater than about 0.5, and frequently are greater than about 1. The log P values of such typical compounds generally are less than about 3.5, often are less than about 3, and sometimes are less than about 2.5. Log P values provide an estimate of the ability of a compound to pass across a diffusion barrier, such as a biological membrane (see for example, Hansch et al., 1968).

The compound, when employed in effective amounts in accordance with the method of the present invention, are selective for the dopamine D₁ receptor, or the dopamine D₁ and D₂ receptors, and do not cause significant activation of receptors associated with undesirable side effects.

The compound, when employed in effective amounts in accordance with the methods described herein, are effective towards providing some degree of prevention of disorders, and/or ameliorate the recurrence of CNS disorders. However, such effective amounts of those compounds are not sufficient to elicit any appreciable side effects, as is demonstrated by decreased effects on preparations believed to reflect effects on the cardiovascular system. As such, administration of compound of the present invention provides a therapeutic window in which treatment of certain CNS disorders is provided, temperature and was left to stir under N₂ overnight. The reaction mixture was washed successively with 2x30 ml of 5% aqueous HCl, 2x30 ml of saturated sodium bicarbonate solution, saturated NaCl solution, and was dried over MgSO₄. After filtration, the filtrate was concentrated under vacuum. Crystallization from diethyl ether gave 5.575 g (69.3%) of the enamide mp 96°-98° C. CIMS (isobutane); M+1 414; ¹H-NMR (CDCl₃); δ 7.59 (d, 2, ArH), 7.46 (m, 3, ArH), 7.35 (m, 3, ArH), 7.20 (d, 2, ArH), 6.60 (s, 1, ArH), 6.45 (s, 1, ArH), 6.18 (s, 1, ArCH), 5.01 (s, 2, ArCH₂ N), 3.80 (S, 3, OCH₃), 3.78 (s, 3, OCH₃), 2.53 (t, 2, ArCH₂), 2.37 (s, 3, ArCH₃), 2.16 (t, 2, CH₂); Anal. (C₂₇ H₂₇ NO₃) C, H, N.

### Trans-2-methyl-6-benzyl-10,11-dimethoxy-5,6,6a,7,8,12b-hexahydro-benzo[a]phen-anthridine-5-one.

A solution of 4.80 g (11.62 mmol) of the 6,7-dimethoxy enamide prepared above, in 500 ml of THF, was introduced to an Ace Glass 500 ml photochemical reactor. This solution was stirred while irradiating for 2 hours with a 450 watt Hanovia medium pressure, quartz, mercury-vapor lamp seated in a water-cooled, quartz immersion well. The solution was concentrated *in vacuo* and crystallized from diethyl ether to provide 2.433 (50.7%) of the 10,11-dimethoxy lactam, mp 183°-195° C. CIMS (isobutane); M+1 414; ¹ H-NMR (CDCl₃); δ 8.13 (d, 1, ArH), 7.30 (s, 1, ArH), 7.23 (m, 6, ArH), 6.93 (s, 1, ArH), 6.63 (s, 1, ArH), 5.38 (d, 1, ArCH₂ N), 5.30 (d, 1, ArCH₂ N), 4.34 (d, 1, Ar₂ CH, J=11.4 Hz), 3.89 (s, 3, OCH₃), 3.88 (s, 3, OCH₃), 3.76 (m, 1, CHN), 2.68 (m, 2, ArCH₂), 2.37 (s, 3, ArCH₃), 2.25 (m, 1, CH₂ CN), 1.75 (m, 1, CH₂ CN); Anal. (C₂₇ H₂₇ NO₃) C, H, N.

### Trans-2-methyl-6-benzyl-10,11-dimethoxy-5,6,6a,7,8,12b-hexahydrobenzo[a]phen-anthridine hydrochloride.

A solution of 1.349 g (3.27 mmol) of the lactam prepared above, in 100 ml dry THF was cooled in an ice-salt bath and 4.0 equiv. (13.0 ml) of 1.0 molar BH₃ was added via syringe. The reaction was heated as reflux under nitrogen overnight. Methanol (10 ml) was added dropwise to the reaction mixture and reflux was continued for 1 hour. The solvent was removed by rotary vacuum evaporation. The residue was chased two times with methanol and twice with ethanol. The flask was placed under high vacuum (0.05 mm Hg) overnight. The residue was dissolved in ethanol and was carefully acidified with concentrated HCl. The violatiles were removed and the product was crystallized from ethanol to afford 1.123 g (78.9%) of the hydrochloride salt, mp 220°-223° C. CIMS (isobutane); M+1 400; ¹H-NMR (CDCl₃, free base); δ 7.37 (d, 2, ArH), 7.33 (m, 2, ArH), 7.26 (m, 1, ArH), 7.22 (s, 1, ArH),

Several ergoline- based molecules such as bromocriptine, lisuride, and pergolide have either been removed from the market or "black-boxed" because they induce cardiac valve pathology. All of these compounds have lightest affinity for D₂ dopamine receptors, and with one exception, they all have but modest efficacy in Parkinson's disease. The exception is apomorphine that is effective as monotherapy, and is the only one of these approved drugs that has high D₁ intrinsic activity. Unfortunately, apomorphine, like dihydrexidine, has a very short duration of action. In addition, its D₂ properties decrease its tolerability (e.g., nausea and emesis).

### Treatment of other CNS Disorders

D₁ receptors are present in high concentration (20 times the density of D₂ receptors) in prefrontal cortex in non-human primates (Lidow et al., 1991), and involved in working memory processes (Sawaguchi and Goldman-Rakic, 1994; Williams and Goldman-Rakic, 1995; Murphy et al., 1996; Zahrt et al., 1997). Optimal stimulation of this brain region is known to potentiate signaling in neurons that are essential to the working memory process (Sawaguchi and Goldman-Rakic, 1991). Lesions of the mesocortical dopamine projection impair working memory performance both in monkeys (Brozoski et al., 1979) and rats (Simon, 1981).
There is strong evidence that D₁ receptor activation may provide cognitive benefits, based on findings that local injection of a D₁ antagonist (but not a D₂ antagonist) 190° C). CIMS (isobutane); M+1 282; ¹H-NMR (DMSO, HCl salt); δ 9.52 (s, 1, NH), 8.87 (d, 2, OH), 7.27 (d, 1, ArH), 7.20 (s, 1, ArH), 7.15 (d, 1, ArH), 6.72 (s, 1, ArH), 6.60 (s, 1, ArH), 4.32 (s, 2, ArCH₂ N), 4.10 (d, 1, ArCH₂ CH, J=11.26 Hz), 2.90 (m, 1, CHN), 2.70 (m, 2, ArCH₂), 2.32 (s, 3, ArCH₃), 2.13 (m, 1, CH₂ CN), 1.88 (m, 1, CH₂ CN); Anal. (C₁₈ H₁₉ NO₂) C, H, N.

Using similar techniques, compounds shown below in Table 1 can be prepared:

**Table 1**

| | | | |
|---|---|---|---|
| 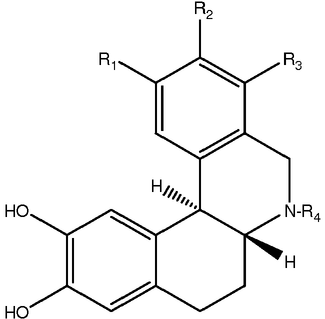 | | | |

| R₁ | R₂ | R₃ | R₄ |
|---|---|---|---|
| CH₃ | H | H | H |
| CH₃ | H | H | CH₃ |
| CH₃ | H | H | C₃H₇ |
| CH₃ | H | CH₃ | C₃H₇ |
| CH₃ | CH₃ | H | H |
| Cl | H | H | H |
| Cl | H | H | C₃H₇ |
| Br | H | H | H |
| Br | H | H | C₃H₇ |
| F | H | H | H |
| F | H | H | C₃H₇ |
| C₂H₅ | H | H | H |
| C₂H₅ | H | H | C₃H₇ |
| C₃H₇ | H | H | H |
| C₃H₇ | H | H | C₃H₇ |

the hypothesis that D₁ agonists have potential in the treatment of cognitive deficits and/or negative symptoms in a variety of conditions including schizophrenia (George et al., 2007; Mu et al., 2007).

### Treatment of Substance Abuse

It has been hypothesized that most, if not all, drugs that are capable of producing dependence increase dopaminergic transmission in specific brain regions, particularly the nucleus accumbens. Appropriate pharmacological agents can be used to treat substance abuse, including abuse of psychostimulants.

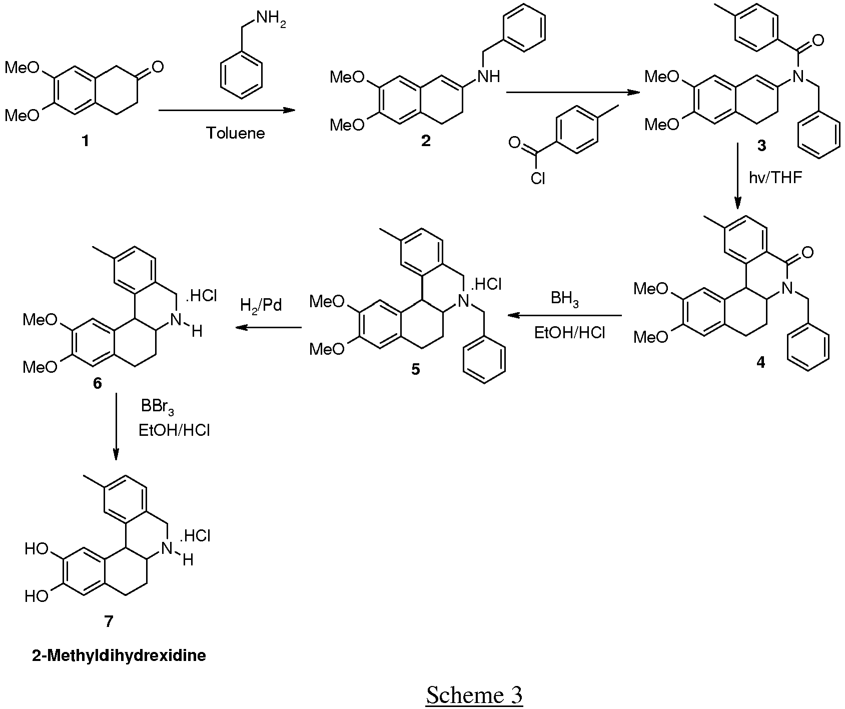

Briefly, a solution of 6,7-dimethoxy-2-tetralone **(1)** in toluene was refluxed with benzylamine with continuous water removal via a Dean-Stark apparatus to yield the crude N-benzyl enamine **(2).** The crude enamine was stirred overnight with *p*-toluoyl chloride and triethylamine in dichloromethane, letting the temperature to rise from 0°C to ambient temperature overnight. The enamide **(3)** thus formed was purified by silica gel column chromatography and subjected to photochemical reaction in THF to yield cyclized product **4.** The ketone **(4)** was reduced by BH₃-THF solution to give **5,** which on de-benzylation by 10% Pd-C catalytic hydrogenation resulted in the formation of *O,O*-dimethyl ether hydrochloride salt **6.** The compound **6** was finally converted into free base and treated with BBr₃ to obtain 2-methyldihydrexidine **(7).** The free base was converted to hydrochloride salt again by EtOH/HCl. The structure of compound **7** was confirmed by NMR and MS data; mp 208-210°C; 'H NMR (DMSO) δ9.53 (s, 1, NH), 9.79 (s, 2, OH), 7.27 (d, 1, ArH, J = 8 Hz), 7.22 (s, 1, ArH), 7.14 (d, 1, ArH, J = 8 Hz), 6.73 (s, 1, ArH), 6.62 (s, 1, ArH), 4.31 (S, 2, ArCH₂N), 4.11 (d, 1, Ar₂CH, J = 11 Hz), 2.90 (m, 1, CHN), 2.70 (m, 2, ArCH₂), 2.32 (s, 3, ArCH₃), 2.16 (m, 1, CHCN), 1.92 (m, 1,CHCN). ESI-MS 282 [M+H]⁺.

### Step 1: N-(4'-Methylbenzoyl)-N-benzyl-6,7-dimethoxy-3,4-dihydro-2-naphthylamine (3)

A solution of 6,7-dimethoxy-2-tetralone **1** (5.0 g, 24.25 mmol) in 150 mL of toluene was stirred at room temperature under a nitrogen atmosphere while 2.85 g (26.66 mmol) of benzylamine is added to the solution. The reaction mixture was heated at reflux overnight under nitrogen with continuous water removal via a Dean-Stark apparatus. The reaction mixture was cooled to room temperature, and the solvent is removed in vacuo to yield the crude N-benzyl enamine **(2)** as a brown oil.

The crude enamine was dissolved in 150 mL of dichloromethane, and the solution was cooled to 0 °C in an ice bath. Triethylamine (1.1 equiv) was added to the solution with stirring. The *p*-toluoyl chloride (4.03 g, 26 mmol) was dissolved in 20 mL of dichloromethane, and this solution was added dropwise to the cold, stirring enamine solution. After complete addition, the ice bath was removed, and the reaction mixture was left to stir overnight at room temperature under a nitrogen atmosphere. The reaction mixture was washed with 2 x 50 mL of 5% HCl, 2 x 50 mL of saturated NaHCO₃ solution, and brine. The organic phase was dried with MgSO₄, filtered, and concentrated. The crude product was passed over a silica gel flash column, eluting with 5% ether in dichloromethane. The collected fractions containing the product were combined and concentrated. The product was crystallized from Et₂O, mp 89-90 °C, (Yield 70%).

### Step 2 : ( + )-trans-2-Methyl-6-benzyl-10,11-dimethoxy-5,6,6a,7,8,12b hexahydrobenzo[a]phenanthridin-5-one (4)

A solution of enamide **3** (3.0g, 7.26 mmol) was prepared in 1L of THF. This solution was placed into an Ace glass 1L photochemical reactor. The solution was stirred while irradiating with a 450 W Hanovia medium pressure, quartz, mercury-vapor lamp seated in a cold tap water-cooled, quartz immersion well. When TLC analysis had indicated the complete disappearance of the starting material (~3 h), the solution was concentrated via rotary evaporation. The product was purified by elution through a silica gel flash column with 5% ether in dichloromethane. The appropriate fractions were combined, and the product was crystallized from diethyl ether, mp 183-185 °C, (Yield 54%).

### Step 3 : ( + )-trans-2-Methyl-6-benzyl-10,11-dimethoxy-5,6,6a,7,8,12b-hexahydrobenzo[a]phenantridine hydrochloride (5)

A solution of **4** (4.0 g, 10.02 mmol) in 200 mL of dry THF is cooled in an ice-salt bath, and 4-5 equiv of a 1.0 M solution of BH₃ in THF was added via syringe. The reaction mixture was heated at reflux until all of the starting material had been consumed (TLC, 5% Et₂O/CH₂Cl₂). Methanol (70 mL) was then added cautiously to the reaction mixture, and the mixture was heated at reflux for an additional 4 h. The reaction mixture was cooled to room temperature, and the solvent was removed in vacuo. Methanol (50 mL) was added to the flask and then removed on the rotary evaporator; this procedure was repeated. Likewise, two 50 mL portions of ethanol were added and removed in the same manner. The reaction flask was then left under high vacuum overnight. The residue was suspended in absolute EtOH and carefully acidified with concentrated HCl. The volatiles were removed, and the product was crystallized from ethanol, mp 220-223 °C, (Yield 80%).

### Step 4 : ( + )-trans-2-Methyl-10,11-dimethoxy-5,6,6a,7,8,12b hexahydrobenzo[a]phenantridine hydrochloride (6)

A solution of 6-N-benzyl hydrochloride salt **5** (2.8 g, 7.01 mmol) in 100 mL of methanol containing 0.60 g of 10% Pd-C catalyst is shaken at room temperature under 50 psi of H₂ overnight. The catalyst is removed by filtration through a pad of celite. The solution is concentrated to dryness on a rotary evaporator, and the product is crystallized from acetonitrile, mp 238-240 °C, (Yield 88%).

### Step 5 : ( + )-trans-2-Methyl-10,11-dihydroxy-5,6,6a,7,8,12b hexahydrobenzo[a]phenantridine hydrochloride (7)

The 0,0-dimethyl ether hydrochloride salt **6** (1.8 g, 5.82 mmol) was converted to their free bases in H₂O with a saturated bicarbonate solution. The aqueous solution was extracted with 3 x 30 mL of dichloromethane. The organic fractions were dried over MgSO₄, filtered, and concentrated in vacuo. The free base was dissolved in 35 mL of dichloromethane, and the solution was cooled to -78 °C. A 1.0 M solution of BBr₃ in dichloromethane (30 mmol, 4-5 equiv) was added slowly to the reaction mixture via syringe. The cooling bath was removed, and the reaction mixture was left to stir under a nitrogen atmosphere overnight, while warming to ambient temperature. Methanol (50 mL) was then added cautiously to the reaction mixture over 20 min. The solvent was removed by rotary evaporation, and the flask was left under high vacuum overnight. The residue was dissolved in water and carefully neutralized to its free base with a saturated bicarbonate solution while cooling in an ice bath. The free base was filtered and washed with cold water. The precipitated free base was dissolved in absolute ethanol, and carefully acidified with concentrated HCl. After removal of the volatiles, the hydrochloride salt was crystallized from MeOH/EtOAc, mp 208-210°C, (Yield 52%).

### EXAMPLE 3: Binding Affinity of Compound 1

The affinity of Compound 1, relative to dihydrexidine, chlorpromazine, and SCH23390, for D₁ and D₂ binding sites was assayed using rat brain striatal homogenates having D₁ and D₂ binding sites labeled with ³H-SCH 23390 and ³H-spiperone, respectively. The data are shown in Table 2.

**Table 2: Dopamine receptor binding affinities of Compound 1^{A}**

| | Rat striatal homogenates | | Cloned human receptors | | | | |
|---|---|---|---|---|---|---|---|
| Drug | D₁ | D₂ | D₁ (C-6) | D_{2L} (C-6) | D₃ (CHO) | D₄ (CHO) | D₅ (HEK) |
| SCH23390 | 0.4 | -nt- | 0.4 | -nt- | -nt- | -nt- | 1.2 |
| chlorpromazine | -nt- | 2.0 | -nt- | 0.74 | 0.86 | 20 | -nt- |
| dihydrexidine | 6.5 | 50 | 2.2 | 180 | 15 | 14 | 14 |
| Compound 1 | 9.0 | 325 | 8.0 | 525 | 95 | 325 | 7.0 |

| | | | | | | | |
|---|---|---|---|---|---|---|---|
| ^{A} All values are **K_{0.5}** in nM concentrations nt = not tested | | | | | | | |

As is well known, the affinity and potency of agonists for members of the GPCR superfamily is affected markedly by receptor expression and by the cellular milieu in which the receptor is expressed. For this reason, rat striatal membranes were used as a standard of comparison. In all of these preparations, Compound 1 was a high affinity full D₁ agonist with a K_{0.5} slightly higher than dihydrexidine. In addition, Compound 1 was somewhat more selective for the D₂ family of receptors than was dihydrexidine (Table 2). It is believed that this compound is D₂ functionally selective, and therefore may decrease nausea that is common with compounds that activate D₂ receptors.

### Affinity of Compound 1 at other receptors:

Compound 1 was tested for its affinity to a host of other potential receptor targets. The strategy used first examined the effects of a 10 µM concentration of Compound 1, and then did full dose-response studies for any receptor in which there was >50% inhibition of binding. For non-dopamine receptors, Compound 1 had little affinity for most receptors that might have been predicted to be engaged by its structural features. This includes various monoamine transporters (DAT, NERT; SERT); many serotonin receptors (5-HT_{1D}; r5-HT_{2A}; 5-HT-_{2B}; 5-HT₃; 5-HT₆; adrenergic receptors (α_{1A}; α_{1B}; α_{2A}; α_{2B}; α_{2C}; rβ₁; rβ₂); hH1; muscarinic receptors (M1; M2; M3; M4; M5); opioids (µ; κ); and a variety of miscellaneous receptors (e.g., EP3; EP4; rPCP; rBZP; etc.). From what is known about the properties of these receptors, there are no obvious concerns about the pharmacological profile of Compound 1.

### EXAMPLE 3: Dopamine D₁ Agonist Activity of Compound 1

The activity of Compound 1 as a dopamine agonist was measured. Studies were done in cloned hD1 receptors expressed in Ltk cells. Compound 1 was a full agonist of similar intrinsic activity and potency when compared to dihydrexidine. The data is shown in Figure 2.

The agonist effects of Compound 1 were completely blocked by the D₁ antagonist SCH23390 (0.3 mg/kg). This data demonstrates the D₁ activity of Compound 1. However, the rotation caused by Compound 1 was not blocked by the D₂ antagonist haloperidol (0.1 mg/kg), which demonstrates the D₁ selectivity over D₂.

### Behavioral effects of Compound 1 in the rat model of Parkinson's disease

The activity of Compound 1 in a unilateral 6-OHDA rat model was measured, and compared to the activity of dihydrexidine. The results are shown in Figure 1. As shown in Figure 1 (top chart), a single subcutaneous injection of 1 mg/kg Compound 1 produced activity lasting for nearly ten hours (N=8). As shown in Figure 1 (bottom chart), dihydrexidine is much shorter acting, with no activity present after three hours (N = 4, consistent with earlier dihydrexidine studies.).

A critical characteristic of a drug for use in Parkinson's disease is that it does not have an overly short duration of action as do dihydrexidine and almost all other full D₁ agonists. One of the reasons for the short duration of action is believed to be the presence of the catechol moiety that is, so far, essential for full agonist actions (Mailman et al., 2001; Mailman and Huang, 2007; Mottola et al., 1996). This makes such compounds metabolically labile by phase 2 drug metabolism. Thus, Compound 1 was selected form a broad genus of hexahydrobenzophenanthridines when it was unexpectedly found to have much better pharmacokinetic properties. This is illustrated in Figures 3A and 3B, with comparison data to dihydrexidine. The duration of action is expected to correlate with peripheral drug levels.

with concentrated HCl. After removal of the volatiles, the HCl salt was crystallized as a solvate from methanol in a yield of 0. 185 g (51%), mp (decomposes @ 190° C). CIMS (isobutane); M+1 282; ¹ H-NMR (DMSO, HCl salt); δ 9.52 (s, 1, NH), 8.87 (d, 2, OH), 7.27 (d, 1, ArH), 7.20 (s, 1, ArH), 7.15 (d, 1, ArH), 6.72 (s, 1, ArH), 6.60 (s, 1, ArH), 4.32 (s, 2, ArCH₂ N), 4.10 (d, 1, ArCH₂ CH, J=11.26 Hz), 2.90 (m, 1, CHN), 2.70 (m, 2, ArCH₂), 2.32 (s, 3, ArCH₃), 2.13 (m, 1, CH₂ CN), 1.88 (m, 1, CH₂ CN); Anal. (C₁₈ H₁₉ NO₂) C, H, N.

Using similar techniques, reference compounds shown below in Table 1 can be prepared:

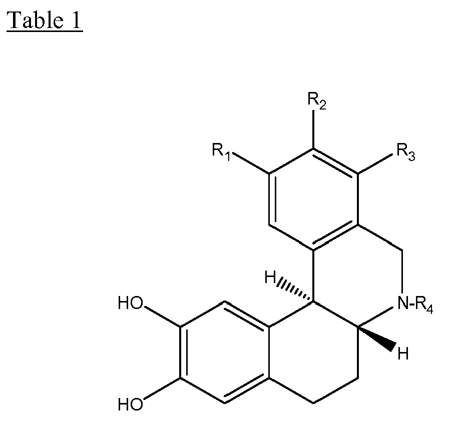

The final volume of the cell suspension recovered from each flask was ca. 14 mL. Scraped membranes from several flasks were then combined. The combined cell suspension was homogenized (10 strokes), 14 mL at a time, using a 15 mL Wheaton Teflon-glass homogenizer. The cell homogenates were combined and spun at 43,000xg (Sorvall RC-5B/SS-34, DuPont, Wilmington, Del.) at 4°C for 20 min. The supernatant was removed, and the pellet was resuspended (10 strokes) in 1 mL of ice cold HOB for each original flask of cells homogenized. This homogenate was then spun again at 43,000xg at 4°C for 20 min. The supernatant was removed and the final pellet was resuspended (10 strokes) in ice cold storage buffer (50 mM Hepes, 6 mM MgCl₂, 1 mM EDTA; pH 7.4) to yield a final concentration of ca. 2.0 mg of protein/mL. Aliquots of the final homogenate were stored in microcentrifuge tubes at -80°C. Prior to their use for adenylate cyclase assays, protein levels for each membrane preparation were quantified using the BCA protein assay reagent (Pierce, Rockford, Ill.) adapted for use with a microplate reader (Molecular Devices; Menlo Park, Calif.).

### Dopamine Receptor Binding Assays

Frozen rat striata were homogenized by seven manual strokes in a Wheaton Teflon-glass homogenizer in 8 mL ice cold 50 mM HEPES buffer with 4.0 mM MgCl₂ (pH 7.4). Tissue was centrifuged at 27,000xg for 10 min, the supernatant was discarded, and the pellet was homogenized (five strokes) and resuspended in ice cold buffer and centrifuged again. The final pellet was suspended at a concentration of 2.0 mg wet weight/mL. The amount of tissue added to each assay tube was 1.0 mg, in a final assay volume of 1.0 mL. D₁ receptors were labeled with [³H]SCH23390 (0.30 nM); D₂ receptors were labeled with [³H]spiperone (0.07 nM); unlabeled ketanserin (50 nM) was added to mask binding to 5-HT₂-type receptors. Total binding was defined as radioligand bound in the absence of any competing drug. Nonspecific binding was estimated by adding unlabeled SCH23390 (1 µM) or unlabeled chlorpromazine (1 µM) for D₁ and D₂ receptor binding assays, respectively. As an internal standard, a competition curve with six concentrations of unlabeled SCH23390 (D₁ binding) or chlorpromazine (D₂ binding) was included in each assay. Triplicate determinations were made for each drug concentration. Assay tubes were incubated at 37°C for 15 minutes, and binding was terminated by filtering with ice cold buffer on a Skatron 12 well cell harvester (Skatron, Inc., Sterling, Va.) using glass fiber filter mats (Skatron no. 7034). Filters were allowed to dry, and 1.0 mL of Optiphase HI-SAF II scintillation fluid was added. Radioactivity was determined on an LKB Wallac 1219 RackBeta liquid scintillation counter (Wallac, Gaithersburg, Md.). Tissue protein levels were estimated using the BCA protein assay reagent (Pierce, Rockford, Ill.).

### Data Analysis for Radioreceptor Assays

Binding data from each assay were analyzed separately. Data were normalized by expressing the average dpm at each competitor concentration as a percentage of total binding. These data were then subjected to nonlinear regression analysis using the algorithm for sigmoid curves in the curve-fitting program InPlot (Graphpad Inc.; San Francisco, Calif.) to generate Ko. values and a Hill coefficient (n_{H}) for each curve. Analysis of the residuals indicated an excellent fit; r values were above 0.99 for all curves in the present experiments.

### Adenylate Cyclase Assay in C-6mD_{1A} Cells

Frozen membranes were thawed and added to assay tubes (10 µg protein/tube) containing a prepared reaction mixture [100 mM Hepes, (pH 7.4), 100 mM NaCl, 4 mM MgCl₂, 2 mM EDTA, 500 µM isobutyl methylxanthine (IBMX), 0.01% ascorbic acid, 10 µM pargyline, 2 mM ATP, 5 µM GTP, 20 mM phosphocreatine, 5 units of creatine phosphokinase (CPK), 1 µM propranolol] and selected drugs. The final reaction volume was 100 µL.

Basal cAMP activity was determined by incubation of tissue in the reaction mixture with no drug added. Tubes were assayed in duplicate and, after a 15 min incubation at 30°C, the reaction was stopped by the addition of 500 µL of 0.1 N HCl. Tubes were vortexed briefly, and then spun in a BHG HermLe Z 230 M microcentrifuge for five min at 15,000xg to precipitate particulates.

### Radioimmunoassay (RIA) of cAMP

The concentration of cAMP in each sample was determined with an RIA of acetylated cAMP, modified from that previously described. Iodination of cAMP was performed using a now-published procedure (Brown et al., 2009). Assay buffer was 50 mM sodium acetate buffer with 0.1% sodium azide (pH 4.75). Standard curves of cAMP were prepared in buffer at concentrations of 2 to 500 fmol/assay tube. To improve assay sensitivity, all samples and standards were acetylated with 10 µl of a 2:1 solution of triethylamine:acetic anhydride. Samples were assayed in duplicate. Each assay tube (total volume 300 µL) contained 25 µL of each sample, 75 µL of buffer, 100 µL of primary antibody (sheep, anti-cAMP, 1:100,000 dilution with 1% BSA in buffer) and 100 µL of [¹²⁵ I]-cAMP (50,000 dpm/100 µL of buffer). Tubes were vortexed and stored at 4° C. overnight (approx. 18 h). Antibody-bound radioactivity was separated by the addition of 25 µL of BioMag rabbit, anti-goat IgG (Advanced Magnetics, Cambridge Mass.), followed by vortexing and incubation at 4°C for 1 h. To these samples 1 mL of 12% polyethylene glycol/50 mM sodium acetate buffer (pH 6.75) was added and tubes were centrifuged at 1700×g for 10 min. Supernatants were aspirated and radioactivity in the pellet was determined using an LKB Wallac gamma counter (Gaithersburg, Md.).

### Data Analysis for Adenylate Cyclase Studies

Data for each sample were expressed initially as pmol/mg/min cAMP. Baseline values of cAMP were subtracted from the total amount of cAMP produced in each drug condition. Data for each drug were expressed relative to the stimulation produced by 100 µM DA.

### References

The following references were cited herein,
Arnsten AF, Cai JX, Murphy BL, Goldman-Rakic PS. 1994. Dopamine D1 receptor mechanisms in the cognitive performance of young adult and aged monkeys. Psychopharmacology (Ber1) 116:143-151.
Asano Y, Yamashita M, Nagai K, Kuriyama M, Yamada K, Tomioka K. 2001. The first asymmetric synthesis of a dopamine D1 agonist, dihydrexidine, employing asymmetric conjugate addition technology. Tetrahedron Letters 42:8493-8495.
Bach ME, Barad M, Son H, Zhuo M, Lu YF, Shih R, Mansuy I, Hawkins RD, Kandel ER. 1999. Age-related defects in spatial memory are correlated with defects in the late phase of hippocampal long-term potentiation in vitro and are attenuated by drugs that enhance the cAMP signaling pathway. Proc Natl Acad Sci U S A 96:5280-5285.
Brown JT, Kant A, Mailman RB. 2009. Rapid, semi-automated, and inexpensive radioimmunoassay of cAMP: Application in GPCR-mediated adenylate cyclase assays. J Neurosci Methods 177:261-266.
Brozoski TJ, Brown RM, Rosvold HE, Goldman PS. 1979. Cognitive deficit caused by regional depletion of dopamine in prefrontal cortex of rhesus monkey. Science 205:929-932.
Cai JX, Arnsten AF. 1997. Dose-dependent effects of the dopamine D1 receptor agonists A77636 or SKF81297 on spatial working memory in aged monkeys. J Pharmacol Exp Ther 283:183-189.
Caine SB, Koob GF, Parsons LH, Everitt BJ, Schwartz JC, Sokoloff P. 1997. D3 receptor test in vitro predicts decreased cocaine self- administration in rats. NeuroReport 8:2373-2377.
Carlsson A. 1959. The occurrence, distribution and physiological role of catecholamines in the nervous system. Pharmacol Rev 11:493.
Carlsson A, Lindqvist M, Magnusson T, WALDECK B. 1958. On the presence of 3-hydroxytyramine in brain. Science 127:471.
Castner SA, Williams GV, Goldman-Rakic PS. 2000. Reversal of antipsychotic-induced working memory deficits by short-term dopamine D1 receptor stimulation. Science 287:2020-2022.
Cederbaum JM, Schleifer LS. 1990. Drugs for Parkinson's Disease, spasticity, and acute muscle spasms. In: Gilman AG, Rall T, Nies A, Taylor P, editors. Goodman & Gilman's The Pharmacological Basis of Therapeutics.Pergamon Press. pp. 463-484.
Cheng Y, Prusoff WH. 1973. Relationship between the inhibition constant (KI) and the concentration of inhibitor which causes 50 per cent inhibition (I50) of an enzymatic reaction. Biochem Pharmacol 22:3099-3108.
Cotzias GC, Papavasiliou PS, Gellene R. 1969. Modification of Parkinsonism--chronic treatment with L-dopa. New England Journal of Medicine 280:337-345.
Ehringer H, Hornykiewicz O. 1960. Verteilung vn Noradrenalin und Dopamin (3-hydroxytyramine) in Gehirn des Menshen und ihr Verhalten bei Evkrankungen des extrapyramidalen systems. Klin. Wschr. Klin Wochenschr 38:1236-1239.
Frey U, Matthies H, Reymann KG, Matthies H. 1991. The effect of dopaminergic D1 receptor blockade during tetanization on the expression of long-term potentiation in the rat CA1 region in vitro. Neurosci Lett 129:111-114.
Hansch C, Hoekman D, Leo A, Zhang L, Li P. 1995. The expanding role of quantitative structure-activity relationships (QSAR) in toxicology. Toxicol Lett 79:45-53.
Hansch C, Steward AR, Anderson SM, Bentley DL. 1968. Parabolic dependence of drug action upon lipophilic character as revealed by a study of hypnotics. J Med Chem 11:1-11.
Haughn Z. Levodopa: life begins at 40? Practical Neurology 6[4], pp. 24-28. 2007. Plymouth Meeting PA, Avondale Medical Publications.
Hornykiewicz O. 1963. Die Topische Lokalisation und das verhalten von noradrenalin und dopamine (3-Hydroxytyramin) in der substantia nigra des normalen und parkinsonkranken menschen. Wien Klin Wochenschr 75:309-312.
Hoyer D, Boddeke HW. 1993. Partial agonists, full agonists, antagonists: dilemmas of definition. Trends Pharmacol Sci 14:270-275.
Huang X, Lawler CP, Lewis MM, Nichols DE, Mailman RB. 2001. D1 dopamine receptors. Int Rev Neurobiol 48:65-139.
Huang YY, Kandel ER. 1995. D1/D5 receptor agonists induce a protein synthesis-dependent late potentiation in the CA1 region of the hippocampus. Proc Natl Acad Sci USA 92:2446-2450.
Jenner P, Demirdemar R. 1997. Dopamine receptor sub-types: from basic sciences to clinical applications. Burke, VA:IOS Press.
Kilts JD, Connery HS, Arrington EG, Lewis MM, Lawler CP, Oxford GS, O'Malley KL, Todd RD, Blake BL, Nichols DE, Mailman RB. 2002. Functional selectivity of dopamine receptor agonists. II. Actions of dihydrexidine in D2L receptor-transfected MN9D cells and pituitary lactotrophs. J Pharmacol Exp Ther 301:1179-1189.
Knoerzer TA, Watts VJ, Nichols DE, Mailman RB. 1995. Synthesis and biological evaluation of a series of substituted benzo[a]phenanthridines as agonists at D1 and D2 dopamine receptors. J Med Chem 38:3062-3070.
Lidow MS, Goldman-Rakic PS, Gallager DW, Rakic P. 1991. Distribution of dopaminergic receptors in the primate cerebral cortex: quantitative autoradiographic analysis using [3H]raclopride, [3H]spiperone and [3H]SCH23390. Neuroscience 40:657-671.
Mailman R, Huang X, Nichols DE. 2001. Parkinson's disease and D1 dopamine receptors. Curr Opin Investig Drugs 2:1582-1591.
Mailman RB. 2007. GPCR functional selectivity has therapeutic impact. Trends Pharmacol Sci 28:390-396.
Mailman RB, Huang X. 2007. Dopamine Receptor Pharmacology. In: Koller W, Melamed E, editors. Handbook of Clinical Neurology (3rd Series) Parkinson's Disease and Related Disorders. Amsterdam:Elsevier. pp. 77-105.
Mailman RB, Nichols DE. 1998. Dopamine D1 receptor agonists as antiparkinson drugs. Trends Pharmacol Sci 19:255-256.
Matthies H, Becker A, Schroeder H, Kraus J, Hollt V, Krug M. 1997. Dopamine D1-deficient mutant mice do not express the late phase of hippocampal long-term potentiation. NeuroReport 8:3533-3535.
Mottola DM, Kilts JD, Lewis MM, Connery HS, Walker QD, Jones SR, Booth RG, Hyslop DK, Piercey M, Wightman RM, Lawler CP, Nichols DE, Mailman RB. 2002. Functional selectivity of dopamine receptor agonists. I. Selective activation of postsynaptic dopamine D2 receptors linked to adenylate cyclase. J Pharmacol Exp Ther 301:1166-1178.
Murphy BL, Arnsten AF, Goldman-Rakic PS, Roth RH. 1996. Increased dopamine turnover in the prefrontal cortex impairs spatial working memory performance in rats and monkeys. Proc Natl Acad Sci U S A 93:1325-1329.
Negash K, Nichols DE. 2001. A new aproach for the synthesis of (+/-)-trans-10,11-dihydroxy-5,6,6a,7,8,12b-Hexahydrobenzo[a]phenanthridine (Dihydrexidine). Tett Lett 37:6971-6972.
Neve KA, Neve RL. 1997. The dopamine receptors. Totowa, N.J.:Humana Press.
Neve KA, Seamans JK, Trantham-Davidson H. 2004. Dopamine receptor signaling. J Recept Signal Transduct Res 24:165-205.
Parkinson J. 1817. An Essay on the Shaking palsy. London:Sherwood, Neely and Jones.
Qandil AM, Miller DW, Nichols DE. 1999. A practical and cost-effective synthesis of 6,7-dimethoxy-2-tetralone. Synthesis 1999:2033-2035.
Sawaguchi T, Goldman-Rakic PS. 1991. D1 dopamine receptors in prefrontal cortex: involvement in working memory. Science 251:947-950.
Sawaguchi T, Goldman-Rakic PS. 1994. The role of D1-dopamine receptor in working memory: local injections of dopamine antagonists into the prefrontal cortex of rhesus monkeys performing an oculomotor delayed-response task. J Neurophysiol 71:515-528.
Schneider JS, Sun ZQ, Roeltgen DP. 1994. Effects of dihydrexidine, a full dopamine D-1 receptor agonist, on delayed response performance in chronic low dose MPTP-treated monkeys. Brain Res 663:140-144.
Sealfon SC, Olanow CW. 2000. Dopamine receptors: from structure to behavior. Trends Neurosci 23:S34-S40.
Self DW, Barnhart WJ, Lehman DA, Nestler EJ. 1996. Opposite modulation of cocaine-seeking behavior by D1- and D2-like dopamine receptor agonists. Science 271:1586-1589.
Simon H. 1981. Dopaminergic A10 Neurons and Frontal System. Journal de Physiologie 77:81-95.
Taylor JR, Lawrence MS, Redmond DE, Jr., Elsworth JD, Roth RH, Nichols DE, Mailman RB. 1991. Dihydrexidine, a full dopamine D1 agonist, reduces MPTP-induced parkinsonism in monkeys. Eur J Pharmacol 199:389-391.
Ungerstedt U, Arbuthnott GW. 1970. Quantitative recording of rotational behavior in rats after 6-hydroxy- dopamine lesions of the nigrostriatal dopamine system. Brain Res 24:485-493.
Urban JD, Clarke WP, von Zastrow M, Nichols DE, Kobilka B, Weinstein H, Javitch JA, Roth BL, Christopoulos A, Sexton PM, Miller KJ, Spedding M, Mailman RB. 2007. Functional selectivity and classical concepts of quantitative pharmacology. J Pharmacol Exp Ther 320:1-13.
Weed MR, Paul IA, Dwoskin LP, Moore SE, Woolverton WL. 1997. The relationship between reinforcing effects and in vitro effects of D1 agonists in monkeys. J Pharmacol Exp Ther 283:29-38.
Weed MR, Vanover KE, Woolverton WL. 1993. Reinforcing effect of the D1 dopamine agonist SKF 81297 in rhesus monkeys. Psychopharmacology (Berl) 113:51-52.
Weed MR, Woolverton WL. 1995. The reinforcing effects of dopamine D1 receptor agonists in rhesus monkeys. J Pharmacol Exp Ther 275:1367-1374.
Williams GV, Castner SA. 2006. Under the curve: critical issues for elucidating D1 receptor function in working memory. Neuroscience 139:263-276.
Williams GV, Goldman-Rakic PS. 1995. Modulation of memory fields by dopamine D1 receptors in prefrontal cortex. Nature 376:572-575.
Zahrt J, Taylor JR, Mathew RG, Arnsten AF. 1997. Supranormal stimulation of D1 dopamine receptors in the rodent prefrontal cortex impairs spatial working memory performance. J Neurosci 17:8528-8535.

Whereas the invention has been has been described herein in reference to specific aspects, features and illustrative embodiments of the invention, it will be appreciated that the utility of the invention is not thus limited.

overnight (approx. 18 h). Antibody-bound radioactivity was separated by the addition of 25 µL of BioMag rabbit, anti-goat IgG (Advanced Magnetics, Cambridge Mass.), followed by vortexing and incubation at 4°C for 1 h. To these samples 1 mL of 12% polyethylene glycol/50 mM sodium acetate buffer (pH 6.75) was added and tubes were centrifuged at 1700×g for 10 min. Supernatants were aspirated and radioactivity in the pellet was determined using an LKB Wallac gamma counter (Gaithersburg, Md.).

### Data Analysis for Adenylate Cyclase Studies

Data for each sample were expressed initially as pmol/mg/min cAMP. Baseline values of cAMP were subtracted from the total amount of cAMP produced in each drug condition. Data for each drug were expressed relative to the stimulation produced by 100 µM DA.

### References

The following references were cited herein.
Arnsten AF, Cai JX, Murphy BL, Goldman-Rakic PS. 1994. Dopamine D1 receptor mechanisms in the cognitive performance of young adult and aged monkeys. Psychopharmacology (Berl) 116:143-151.
Asano Y, Yamashita M, Nagai K, Kuriyama M, Yamada K, Tomioka K. 2001. The first asymmetric synthesis of a dopamine D1 agonist, dihydrexidine, employing asymmetric conjugate addition technology. Tetrahedron Letters 42:8493-8495.
Bach ME, Barad M, Son H, Zhuo M, Lu YF, Shih R, Mansuy I, Hawkins RD, Kandel ER. 1999. Age-related defects in spatial memory are correlated with defects in the late phase of hippocampal long-term potentiation in vitro and are attenuated by drugs that enhance the cAMP signaling pathway. Proc Natl Acad Sci U S A 96:5280-5285.

## Claims

1. Use of a pharmaceutical composition for the manufacture of a medicament for treating a disorder selected from the group consisting of Parkinson's disease, parkinsonism, restless leg syndrome, schizophrenia, substance abuse, Huntington's chorea, tardive dyskinesia, mood and anxiety disorders, Tourette's syndrome, neurodegeneration resulting from acute events like stroke, and renal, or pulmonary dysfunction, and wherein the composition has a duration of action sufficient for administration no more than three-times daily and that causes typical and/or functionally selective activation of one of more dopamine receptors for oral administration comprising a compound of the formula: 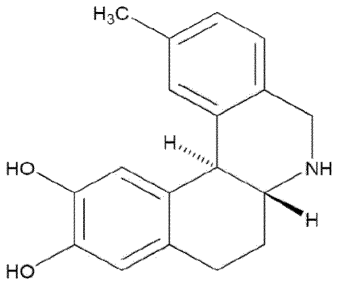 and a pharmaceutically acceptable carrier, wherein the compound is present in an amount of 0.5 to 5 mg/kg.

2. Use of the pharmaceutical composition of Claim 1 wherein the composition is in the form of an oral disintegrating tablet.

3. Use of a compound of the formula: 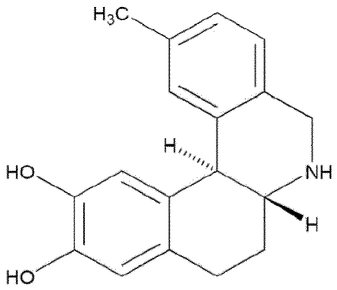 in the preparation of a medicament for treating a dopamine-related dysfunction of the central nervous system **characterized by** an apparent neurological, psychiatric physiological, psychological, or behavioral disorder in a patient suffering said CNS dysfunction,
wherein the composition has a duration of action sufficient for administration no more than three-times daily and that causes typical or functionally selective activation of one of more dopamine receptors,
wherein the compound is present in an amount of 0.5 to 5 mg/kg,
wherein the composition is in the form of oral disintegrating tablet, wherein the disorder is selected from the group consisting of Parkinson's disease, parkinsonism, restless leg syndrome, schizophrenia, substance abuse, Huntington's chorea, tardive dyskinesia, mood and anxiety disorders, Tourette's syndrome, neurodegeneration resulting from acute events like stroke, and renal or pulmonary dysfunction.

4. Use of a pharmaceutical composition for the manufacture of a medicament for use in treating a disorder selected from the group consisting of Parkinson's disease, parkinsonism, restless leg syndrome, schizophrenia, substance abuse, Huntington's chorea, tardive dyskinesia, mood and anxiety disorders, Tourette's syndrome, neurodegeneration resulting from acute events like stroke, and renal, or pulmonary dysfunction, and wherein the composition has a duration of action sufficient for administration no more than three-times daily and that causes typical and/or functionally selective activation of one of more dopamine receptors,
comprising a compound of the formula: 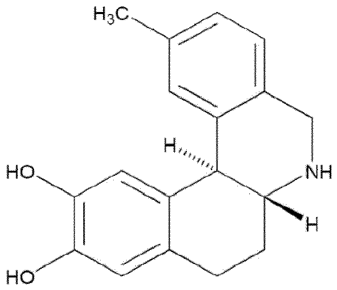 and a pharmaceutically acceptable carrier, wherein the compound is present in an amount of 0.5 to 5 mg/kg, and wherein the composition is in the form of an intranasal formulation or transdermal formulation.
for use in treating a disorder selected from the group consisting of Parkinson's disease, parkinsonism, restless leg syndrome, schizophrenia, Alzheimer's disease and other disorders with cognitive deficits (including age associated cognitive deficits), substance abuse, autism, Huntington's chorea, tardive dyskinesia, attention deficit hyperactivity disorder and related developmental disorders, mood and anxiety disorders, Tourette's syndrome, neurodegeneration resulting from acute events like stroke, and renal, or pulmonary dysfunction, and wherein the composition has a duration of action sufficient for administration no more than three-times daily and that causes typical and/or functionally selective activation of one of more dopamine receptors.

5. Use of a compound of the formula: 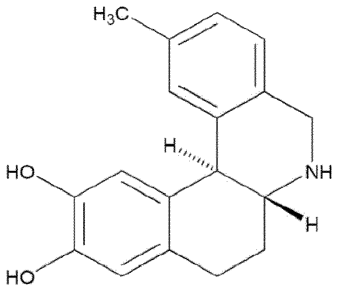 in the preparation of a medicament for treating a dopamine-related dysfunction of the central nervous system **characterized by** an apparent neurological, psychiatric physiological, psychological, or behavioral disorder in a patient suffering said CNS dysfunction, wherein the composition has a duration of action sufficient for administration no more than three-times daily and that causes typical or functionally selective activation of one of more dopamine receptors,
wherein the composition is in the form of a transdermal formulation, or an intranasal formulation,
wherein the disorder is selected from the group consisting of Parkinson's disease, parkinsonism, restless leg syndrome, schizophrenia, substance abuse, Huntington's chorea, tardive dyskinesia, mood and anxiety disorders, Tourette's syndrome, neurodegeneration resulting from acute events like stroke, and renal or pulmonary dysfunction.

## Patentansprüche

1. Verwendung einer pharmazeutischen Zusammensetzung zur Herstellung eines Arzneimittels zum Behandeln einer Störung ausgewählt aus der Gruppe bestehend aus Parkinson-Krankheit, Parkinsonismus, Restless-Leg-Syndrom, Schizophrenie, Substanzmissbrauch, Huntington-Chorea, tardiver Dyskinesie, Gemüts- und Angststörungen, Tourette-Syndrom, Neurodegeneration, resultierend von akuten Ereignissen wie Schlaganfall, und Nieren- oder Lungenfunktionsstörungen, und wobei die Zusammensetzung eine Wirkungsdauer besitzt, die zur Verabreichung von nicht mehr als dreimal täglich ausreicht und die typische und/oder funktionell selektive Aktivierung von einem von mehreren Dopamin-Rezeptoren verursacht, zur oralen Verabreichung, beinhaltend eine Verbindung der Formel: 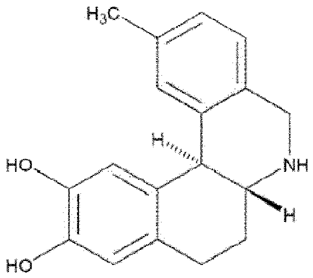 und einen pharmazeutisch akzeptablen Träger, wobei die Verbindung in einer Menge von 0,5 bis 5 mg/kg präsent ist.

2. Verwendung der pharmazeutischen Zusammensetzung gemäß Anspruch 1, wobei die Zusammensetzung in der Form einer oral zerfallenden Tablette vorliegt.

3. Verbindung einer Verbindung der Formel: 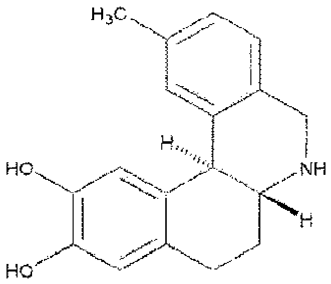 in der Zubereitung eines Arzneimittels zum Behandeln einer Dopamin-bezogenen Funktionsstörung des zentralen Nervensystems, **gekennzeichnet durch** eine scheinbare neurologische, psychiatrisch-physiologische, psychologische oder verhaltensmäßige Störung bei einem Patienten, der an der ZNS-Funktionsstörung leidet,
wobei die Zusammensetzung eine Wirkungsdauer besitzt, die zur Verabreichung von nicht mehr als dreimal täglich ausreicht und die typische oder funktionell selektive Aktivierung von einem von mehreren Dopamin-Rezeptoren verursacht,
wobei die Verbindung in einer Menge von 0,5 bis 5 mg/kg präsent ist,
wobei die Zusammensetzung in der Form einer oral zerfallenden Tablette vorliegt, wobei die Störung ausgewählt ist aus der Gruppe bestehend aus Parkinson-Krankheit, Parkinsonismus, Restless-Leg-Syndrom, Schizophrenie, Substanzmissbrauch, Huntington-Chorea, tardiver Dyskinesie, Gemüts- und Angststörungen, Tourette-Syndrom, Neurodegeneration, resultierend von akuten Ereignissen wie Schlaganfall, und Nieren- oder Lungenfunktionsstörungen.

4. Verwendung einer pharmazeutischen Zusammensetzung zur Herstellung eines Arzneimittels zur Verwendung beim Behandeln einer Störung ausgewählt aus der Gruppe bestehend aus Parkinson-Krankheit, Parkinsonismus, Restless-Leg-Syndrom, Schizophrenie, Substanzmissbrauch, Huntington-Chorea, tardiver Dyskinesie, Gemüts- und Angststörungen, Tourette-Syndrom, Neurodegeneration, resultierend von akuten Ereignissen wie Schlaganfall, und Nieren- oder Lungenfunktionsstörungen, und wobei die Zusammensetzung eine Wirkungsdauer besitzt, die zur Verabreichung von nicht mehr als dreimal täglich ausreicht und die typische und/oder funktionell selektive Aktivierung von einem von mehreren Dopamin-Rezeptoren,
beinhaltend eine Verbindung der Formel: 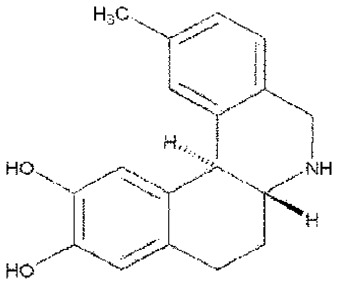 und einen pharmazeutisch akzeptablen Träger, wobei die Verbindung in einer Menge von 0,5 bis 5 mg/kg präsent ist, und wobei die Zusammensetzung in der Form einer intranasalen Formulierung oder transdermalen Formulierung vorliegt.
zur Verwendung beim Behandeln einer Störung ausgewählt aus der Gruppe bestehend aus Parkinson-Krankheit, Parkinsonismus, Restless-Leg-Syndrom, Schizophrenie, Alzheimer-Krankheit und anderen Störungen mit kognitiven Defiziten (einschließlich altersassoziierten kognitiven Defiziten), Substanzmissbrauch, Autismus, Huntington-Chorea, tardiver Dyskinesie, Aufmerksamkeitsdefizit-/Hyperaktivitätsstörung und verwandten Entwicklungsstörungen, Gemüts- und Angststörungen, Tourette-Syndrom, Neurodegeneration, resultierend von akuten Ereignissen wie Schlaganfall, und Nieren- oder Lungenfunktionsstörungen, und wobei die Zusammensetzung eine Wirkungsdauer besitzt, die zur Verabreichung von nicht mehr als dreimal täglich ausreicht und die typische und/oder funktionell selektive Aktivierung von einem von mehreren Dopamin-Rezeptoren.

5. Verbindung einer Verbindung der Formel: 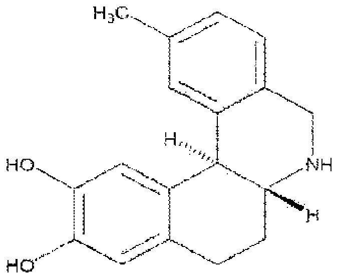 in der Zubereitung eines Arzneimittels zum Behandeln einer Dopamin-bezogenen Funktionsstörung des zentralen Nervensystems, **gekennzeichnet durch** eine scheinbare neurologische, psychiatrisch-physiologische oder verhaltensmäßige Störung bei einem Patienten, der an der ZNS-Funktionsstörung leidet, wobei die Zusammensetzung eine Wirkungsdauer besitzt, die zur Verabreichung von nicht mehr als dreimal täglich ausreicht und die typische oder funktionell selektive Aktivierung von einem von mehreren Dopamin-Rezeptoren verursacht,
wobei die Zusammensetzung in der Form einer transdermalen Formulierung oder einer intranasalen Formulierung vorliegt,
wobei die Störung ausgewählt ist aus der Gruppe bestehend aus Parkinson-Krankheit, Parkinsonismus, Restless-Leg-Syndrom, Schizophrenie, Substanzmissbrauch, Huntington-Chorea, tardiver Dyskinesie, Gemüts- und Angststörungen, Tourette-Syndrom, Neurodegeneration, resultierend von akuten Ereignissen wie Schlaganfall, und Nieren- oder Lungenfunktionsstörungen.

## Revendications

1. Utilisation d'une composition pharmaceutique pour la fabrication d'un médicament pour traiter un trouble choisi dans le groupe constitué de la maladie de Parkinson, du parkinsonisme, du syndrome des jambes sans repos, de la schizophrénie, de la toxicomanie, de la chorée de Huntington, de la dyskinésie tardive, des troubles de l'humeur et de l'anxiété, du syndrome de la Tourette, de la neuro- dégénérescence résultant d'événements aigus comme un accident vasculaire cérébral et une insuffisance rénale ou pulmonaire, et dans lequel la composition a une durée d'action suffisante pour une administration ne dépassant pas trois fois par jour et provoquant l'activation typique et/ou fonctionnellement sélective d'un ou plusieurs récepteurs de la dopamine pour une administration orale comprenant un composé de formule : 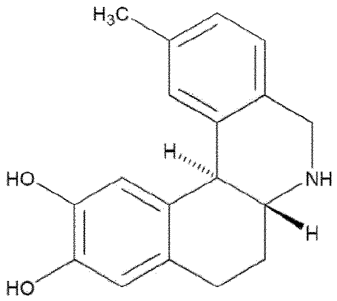 et un support pharmaceutiquement acceptable, dans lequel le composé est présent en une quantité de 0,5 à 5 mg/kg.

2. Utilisation de la composition pharmaceutique selon la revendication 1, dans laquelle la composition est sous forme de comprimé à désintégration orale.

3. Utilisation d'un composé de la formule : 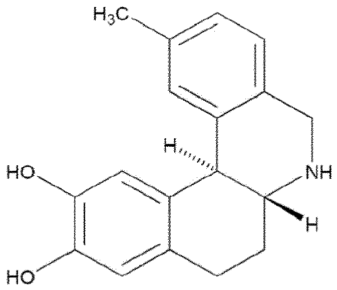 dans la préparation d'un médicament pour le traitement d'un dysfonctionnement lié à la dopamine du système nerveux central **caractérisé par** un trouble apparent neurologique, psychiatrique physiologique, psychologique ou du comportement chez un patient souffrant dudit dysfonctionnement SNC,
dans lequel la composition a une durée d'action suffisante pour une administration ne dépassant pas trois fois par jour et provoquant l'activation typique ou fonctionnellement sélective d'un ou plusieurs récepteurs de la dopamine,
dans lequel le composé est présent en une quantité de 0,5 à 5 mg/kg,
dans lequel la composition est sous forme de comprimé à dissolution orale, dans laquelle le trouble est choisi dans le groupe constitué de la maladie de Parkinson, du parkinsonisme, du syndrome des jambes sans repos, de la schizophrénie, de la toxicomanie, de la chorée de Huntington, de la dyskinésie tardive, des troubles de l'humeur et de l'anxiété, du syndrome de la Tourette, de la neurodégénérescence résultant d'événements aigus comme un accident vasculaire cérébral et une insuffisance rénale ou pulmonaire.

4. Utilisation d'une composition pharmaceutique pour la fabrication d'un médicament pour traiter un trouble choisi dans le groupe constitué de la maladie de Parkinson, du parkinsonisme, du syndrome des jambes sans repos, de la schizophrénie, de la toxicomanie, de la chorée de Huntington, de la dyskinésie tardive, des troubles de l'humeur et de l'anxiété, du syndrome de la Tourette, de la neurodégénérescence résultant d'événements aigus comme un accident vasculaire cérébral et une insuffisance rénale ou pulmonaire, et dans lequel la composition a une durée d'action suffisante pour une administration ne dépassant pas trois fois par jour et provoquant l'activation typique et/ou fonctionnellement sélective d'un ou plusieurs récepteurs de la dopamine,
comprenant un composé de la formule : 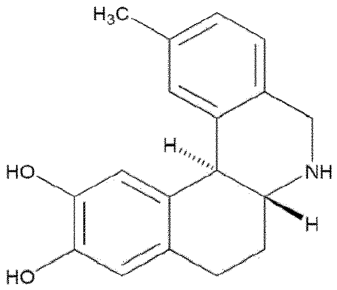 et un support pharmaceutiquement acceptable, dans lequel le composé est présent en une quantité de 0,5 à 5 mg/kg et dans lequel la composition est sous forme d'une formulation intranasale ou d'une formulation transdermique,
pour utilisation dans le traitement d'un trouble choisi dans le groupe constitué de la maladie de Parkinson, du syndrome des jambes sans repos, de la schizophrénie, de la maladie d'Alzheimer et autres troubles avec des déficits cognitifs (y compris les déficits cognitifs associés à l'âge), de la toxicomanie, de l'autisme, de la chorée de Huntington, de la dyskinésie tardive, du trouble déficitaire de l'attention avec hyperactivité et troubles du développement associés, des troubles de l'humeur et de l'anxiété, du syndrome de la Tourette, de la neurodégénérescence résultant d'événements aigus comme un accident vasculaire cérébral et une insuffisance rénale ou pulmonaire, et dans lequel la composition a une durée d'action suffisante pour une administration ne dépassant pas trois fois par jour et provoquant l'activation typique et/ou fonctionnellement sélective d'un ou plusieurs récepteurs de la dopamine.

5. Utilisation d'un composé de la formule : 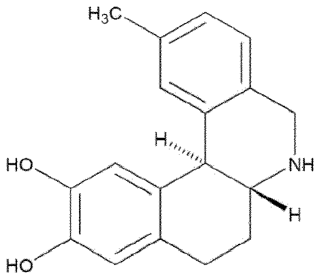 dans la préparation d'un médicament pour le traitement d'un dysfonctionnement lié à la dopamine du système nerveux central **caractérisé par** un trouble apparent neurologique, psychiatrique physiologique, psychologique ou du comportement chez un patient souffrant dudit dysfonctionnement SNC, et dans lequel la composition a une durée d'action suffisante pour une administration ne dépassant pas trois fois par jour et provoquant l'activation typique et/ou fonctionnellement sélective d'un ou plusieurs récepteurs de la dopamine,
et dans lequel la composition est sous forme d'une formulation intranasale ou d'une formulation transdermique,
dans lequel le trouble est choisi dans le groupe constitué de la maladie de Parkinson, du parkinsonisme, du syndrome des jambes sans repos, de la schizophrénie, de la toxicomanie, de la chorée de Huntington, de la dyskinésie tardive, des troubles de l'humeur et de l'anxiété, du syndrome de la Tourette, de la neurodégénérescence résultant d'événements aigus comme un accident vasculaire cérébral et une insuffisance rénale ou pulmonaire.
